(19)

**Europäisches Patentamt**

**European Patent Office**

**Office européen des brevets**

(11) **EP 4 560 647 A1**

(12) **EUROPEAN PATENT APPLICATION**

(43) Date of publication:
**28.05.2025 Bulletin 2025/22**

(21) Application number: **23211490.0**

(22) Date of filing: **22.11.2023**

(51) International Patent Classification (IPC):
**G16H 40/63** (2018.01)  **G06N 20/00** (2019.01)
**G16H 30/00** (2018.01)  **G10L 15/00** (2013.01)
**A61B 5/00** (2006.01)

(52) Cooperative Patent Classification (CPC):
**G16H 30/20; G06N 20/00; G16H 10/60;**
**G16H 20/00; G16H 30/40; G16H 40/67;**
**G16H 50/20; G16H 50/70;** A61B 5/00; G10L 15/00

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventor: **HAHN, Artur**
**Eindhoven (NL)**

(74) Representative: **Philips Intellectual Property &**
**Standards**
**High Tech Campus 52**
**5656 AG Eindhoven (NL)**

(54) **MACHINE LEARNING ASSISTED IMAGING OPERATIONS**

(57)  System and related method for facilitating imaging operation of a medical imaging apparatus- The system comprises an input interface (IN) capable for receiving input data pertaining to an intended imaging operation in relation to a patient. A trained machine learning model (M) having natural language processing capability processes the input data into output data indicative of an imaging instruction for the said imaging operation. An output interface (OUT) provides the output data, thereby facilitating the imaging operation.

The system fosters more efficient operation of the medical imaging apparatus.

**FIG. 3**

**Description**

FIELD OF THE INVENTION

The invention relates to a system for facilitating imaging operation of a medical imaging apparatus, to an imagining arrangement including such a system, to a related method, to a training system for training a machine learning model for use in such a system, to a related machine learning training method, to a training data provider system capable of providing data for the training system, to a computer program element and to a computer readable medium, and to as use of machine learning model.

BACKGROUND OF THE INVENTION

Medical imaging is a first-line mainstay of medicine, at least since discovery of X-rays by Wilhelm Roentgen in the 19th century. Being able to see "inside" a patient without intervention is invaluable for diagnosis, therapy, panning and other medical tasks.

Medical imaging equipment such as CT (computed tomography) or MRI (magnetic resonance imaging) is however complex machinery. Its correct operation to achieve the imaging quality needed of the task at hand is no mean feat. There may be many different settings, parameters, adjustments, etc that command mastery.

Even experienced operators may get it wrong, at times, be it due to time pressure, fatigue, etc.

SUMMARY OF THE INVENTION

There may therefore be a need for improved, efficient imaging or handling of imaging equipment, in particular in the medical field.

An object of the present invention is achieved by the subject matter of the independent claims where further embodiments are incorporated in the dependent claims. It should be noted that the following described aspect of the invention equally applies to the imagining arrangement including such a system for facilitating imaging operation, to the related method, to the training system for training the machine learning model for use in such system, to the related machine learning training method, to the training data provider system capable of providing data for the training system, to the computer program element and to the computer readable medium.

In one aspect there is provided a system for facilitating imaging operation of a medical imaging apparatus, comprising:

an input interface capable for receiving input data pertaining to an intended imaging operation in relation to a patient;
a trained machine learning model having natural language processing capability and capable of processing the input data into output data indicative of an imaging instruction for the said imaging operation, and
an output interface capable of providing the output data, thereby facilitating the imaging operation.

The request may be in fee-text or speech or any other from, and merely outlines the imaging task one wishes to achieve. No reference in such request need be made to any imaging instructions as such, parameters, etc, as pertains to the imaging apparatus. Thus, the system allows ergonomic, efficient, user friendly, operation of imaging apparatus by user. The user merely specifies in the request what the imaging should achieve. The system then returns the corresponding instructions that represent information on how this imaging task is to be achieved with the imager.

In embodiments, the imaging instruction is any one of i) machine-sided and ii) patient-sided,

wherein the machine-sided imaging instruction includes one or more machine settings based on which one or more components of the imaging apparatus are controllable to thereby effect, at least in parts, the said imaging operation, and
wherein the patient-sided imaging instruction includes one or more instructions for the patient behavior in relation to the imaging operation.

In embodiments, the said patient-sided instruction includes one or more instructions for patient to assume a pose relative to acquisition equipment of the imaging apparatus), and/or for patient to perform a bio-activity in a particular manner.

In embodiments, the output interface capable of interfacing with circuitry of the imaging apparatus thereby facilitating the controlling the said one or more components.

In embodiments, the model is based on at least in parts on an artificial neural network architecture.

In embodiments, the model is of the generative type. The model may be capable of autoregressive processing of the input or of intermediate data.

In embodiments, the model is capable of sequence-to-sequence processing.

In embodiments, the model includes a natural language processing component capable of processing the input data into one or more intermediate natural language representations, and a down-stream component capable of processing the one or more intermediate natural language representation into the said output data. The intermediate natural language representations may be a latent of hidden state/data within the model. The model may have an encoder-decoder structure.

In embodiments, the down-stream component is any one of: a diffusion model, a decision tree, a decoder architecture.

In embodiments, the model includes an attention mechanism capable of coding for context as per the input data and/or with respect to intermediate data. Specifically, in embodiments, the attention mechanism is capable of coding for context as per different portions in the input data and/or in intermediate data.

Intermediate data may relate to data at different locations in a data processing flow as caused by the model when processing the input data, such as in models having a cascaded processing flow structure, such as models having layers as in artificial neural networks. The attention mechanism may itself be arranged as such layer, optionally alternating with other layers, such as layers that from a neural network, such as fully connected or convolutional layers.

Such context coding may include capability of expression of different relationships between portion(s) of input data to other portion(s) of the input data, or between different portions of the input data and intermediate data as generated by model when processing the input. Such context may also code for expression of relationship between portions within intermediate data, or between different items of input data or intermediate data at different locations in a data processing flow within the model, such as at different layers in a neural network type model. The different portions may relate to different sequential elements, such as different tokens at different positions, etc, in the input and/or in intermediate data. The relationships may be so expressed in scores (numbers) that vary with the level or such relationship, such as how closely such portions are related to each other, with different pairs of portions having a different level of such relationship, eg, closeness of such relatedness. Such attention mechanism allows more efficient processing of in particular sequential data in deployment and/or training of model.

In embodiments, the input data is indicative of a particular make and model of the imaging apparatus, and wherein the output data is specific to the said particular make and model of the imaging apparatus.

In embodiments, the input data includes sensor data of the patient as capturable by a sensor. Such sensor, such as a vide or still image may be installed in the exam room with the imager.

In embodiments, the said model previously trainable in at least two phases, in a pretraining phase using first training data, and in a subsequent further training phase using second training data.

In embodiments, the first and second training data represent medical knowledge, with one being more general than the other.

In embodiments, the medical data in the first training data relates to general medical knowledge, whereas the medical data in the second training data relates to medical imaging knowledge.

In another aspect there is provided a training system capable of pre-training and/or training, based on respective pre-training/training data, the system's model of any one of the above-mentioned embodiment.

In another aspect there is provided a training data provider system capable of providing the pre-training/training data for the training system.

In another aspect there is provided a method of facilitating imaging operation of a medical imaging apparatus, comprising:

receiving input data pertaining to an intended imaging operation in relation to a patient;
with a trained machine learning model having natural language processing capability, processing the input data into output data indicative of an imaging instruction for the said imaging operation, and
providing the output data, thereby facilitating the imaging operation.

In yet another aspect there is provided a method of pretraining and/or training, based on respective pre-training/training data, the model in the system or method of any one of the above mentioned aspects or embodiments.

In yet another aspect there is provided a method of providing the training data for the (pre-)training method.

In yet another aspect there is provided a computer program element, which, when being executed by at least one computing system, is adapted to cause the at least one computing system to perform a method as per any one of the above mentioned aspects.

In yet another aspect there is provided at least one computer readable medium having stored thereon the program element, or having stored thereon the machine learning model as per in any one of the above mentioned aspects or embodiments.

In yet another aspect there is provided a use of a trained machine learning model capable of natural language processing for generation of at least one imaging instruction for operation of an imaging apparatus for an imaging task.

What is proposed herein in some embodiments is to use a language model trained on medical data for generation of imaging instructions for an imaging apparatus in pursuit of an imaging task in respect of a given patient.

The input imaging request information can be provided in (natural language) text form, in particular in free text form, in tabular form, which the user may provide via a suitable interface. However, graphics input is not excluded herein. In addition, or instead of text, or in addition thereof, the user can provide audio instructions or video as need be. In such cases, the facilitator system may include, or may interface with, suitable transducers that capture speech and/or video and converts same into suitable data format for processing. Natural language recognition may be used. In a similar manner, the output instructions can be provided in text, audio, video as needed, or in a combination of any two or more of the above formats.

In particular output of model may be provided in a format comprising strings of symbols other than, or in addition to, natural language text. Structured output such as in XML, or in other may be provided as specifications as output. For example, the output may comprise strings of imaging command, etc, in a form/format compatible with the imaging apparatus ("imager") to be operated. Such commands or other suitable parameters/specifications in such structured format may then be used by control interface(s) of the imaging apparatus to effect the imaging task so requested.

The imaging request can be provided by the user in a mere descriptive, high level, "phenomenological" manner using natural language. This imaging request is then translated by the model into imaging instructions at different levels of generality, even down to the specific make and/or model of the imager to be used. This greatly reduces everyday operational complexity. It allows even medical "newbies" (junior doctors, etc) to operate imaging equipment safely and with good, clinically usable results. And even seasoned imaging pundits can benefit as the proposed setup may increase imaging throughput, a welcome effect in busy, often understaffed, clinical environments. It is proposed herein to harness machine learning models, in particular language models, for imaging facilitation purposes, especially in the medical field. The natural language model by be combined with other one or more models, upstream or downstream the model, thus forming an ML processing pipeline. Some components/models in this pipeline may be non-ML based.

"user" relates to a person, such as medical personnel or other, operating the imaging apparatus or overseeing the imaging procedure. In other words, the user is in general not the patient who is to be imaged. However, in some application scenarios, the model's output may also be provided to the patient, as will be explained below in the relevant embodiments.

In general, the term *"machine learning"* includes a computerized arrangement (or module) that implements a machine learning ("ML") algorithm. Some such ML algorithms operate to adjust a machine learning model, thus configuring same to perform ("learn") a task. Other ML operate direct on training data, not necessarily using such an explicit model, thus in such cases the training data may form the model, or may be part of such model. This adjusting or updating of the model is called "training". In general, task performance by the ML model may improve measurably with training experience. Training experience may include suitable training data, and exposure of the model to such data. Task performance may improve, the better the data represents the task to be learned. Training experience helps improve performance if the training data well represents a distribution of examples over which the final system performance is measured. See for example, T. M. Mitchell, "Machine Learning", page 2, section 1.1, page 6 1.2.1, McGraw-Hill, 1997. The performance may be measured by objective tests based on output produced by the model in response to feeding the model with test data. The performance may be defined in terms of a certain error rate to be achieved for the given test data.

BRIEF DESCRIPTION OF THE DRAWINGS

Exemplary embodiments of the invention will now be described with reference to the following drawings, which, unless stated otherwise, are not to scale, wherein:

Fig. 1 shows a schematic block diagram of a medical imaging arrangement;
Fig. 2 shows a component pipeline for an imaging operation of an imaging apparatus;
Fig. 3 shows a block diagram of a facilitator system as may be used to operate an imaging apparatus;
Fig. 4 shows a block diagram of an architecture of a machine learning model as may be used in embodiments herein;
Fig. 5 shows a training system for training a machine learning model based on training data;
Fig. 6 shows a flow chart of a computer-implemented method of facilitating operation or of an imaging apparatus; and
Fig. 7 shows a flow chart of a computer-implemented method of training a machine learning model based on training data.

DETAILED DESCRIPTION OF EMBODIMENTS

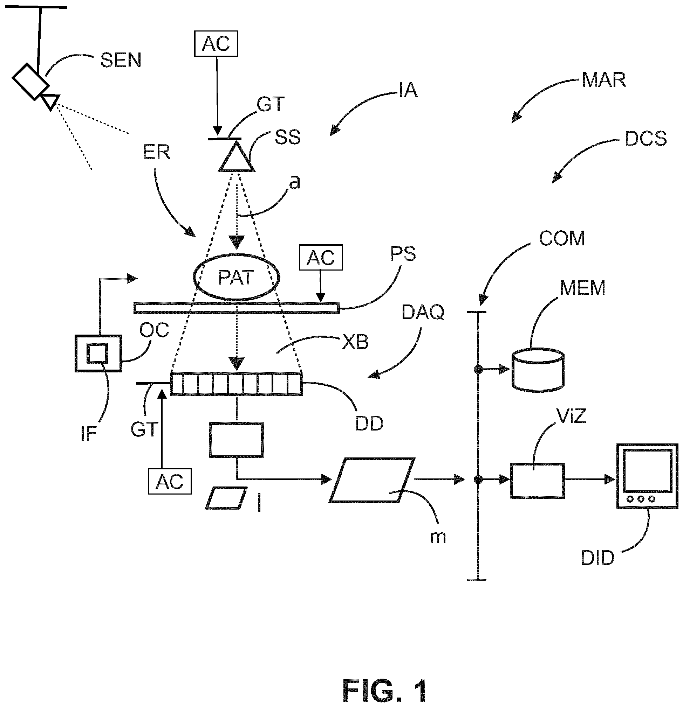

Reference is now made to the block diagram of Fig. 1 which shows a medical imaging arrangement MAR. Broadly, the arrangement MAR includes a preferably medical imaging apparatus IA. The imaging apparatus IA (for brevity referred to herein as "imager") is operative, in one or more imaging sessions, to obtain medical imagery *m*. The medical imagery *m* so obtained may be passed through wired, wireless, or hybrid, telecommunication network COM, or in any other way to a data consumer DCS. More "hands-on" data transfers options such as via removable storage, dongles, etc, are not excluded herein.

The data consumer section DCS may include memory MEM on which the imagery M is stored, such as for later reference, radiological review ("reading"), etc. A visualizer VIZ may be operative to produce visualizations of imagery which may be displayed on a display device DID. The imagery may inform therapy planning and/or diagnosis in a medical setting. The imagery may be reviewed by a reviewer such as a radiologist or other medical professional. Other data consumers in section DSC may include an image post-processing module, such as for segmentation, annotation, etc (not shown). Whilst in the present main reference is made to the medical domain, this is not necessarily at the exclusion of applications in other domains.

The medical imagery *m* obtained by the imager IA is preferably representative of internal structures, organs, etc, of the patient PAT who is to be imaged in an image session by imaging apparatus IA. Thus, the medical imagery allows for a non-invasive manner in which to "look" inside the patient, and to so inform diagnostic, therapeutic, planning strategies among other application scenarios.

Broadly, and as will be explained in more detail below, what is proposed herein is an imaging facilitator IF, a computing arrangement, that allows a user to easily obtain imaging instructions for use with the current patient PAT and imager IA, and an intended imaging task at hand. Thanks to the imaging facilitator IF, even non-specialist user may be able to operate the imager IA safely and efficiently to obtain imagery *m* of sufficient image quality.

With more detailed reference to the imaging apparatus IA, this includes a data acquisition unit DAQ that is operable to acquire measurements $\lambda$ during the imaging session with suitable detector device DD equipment. The measurements $\lambda$ may include variations of a measurement signal to which the patient is exposed to. The measurement signal is thought to correlate with spatial or functional features of structures of interest (also referred to as region of interest (ROI) ) within the patient. The measurement may include intensity measurements $\lambda$, eg projection raw data, such as may be utilized in computed tomography or radiography, as needed.

Before proving more in-depth detail on the imaging facilitator IF, more detailed reference is first made to the imaging apparatus, its components and the imaging context more generally, in order to so better assist the later, more detail explanations on the functioning of the said imaging facilitator IF.

Broadly, the imaging apparatus includes a signal source SS and the detection device DD.

The signal source SS generates a signal, for example an interrogating signal, which interacts with the patient to produce a response signal which is then measured by the detector device DD and converted into the measurement data $\lambda$ such as the said medical imagery.

One example of the imager IA is an X-ray based imaging apparatus such as a radiography apparatus, configured to produce protection imagery. Volumetric tomographic (cross sectional) imaging is not excluded herein, such as via a C-arm imager or a CT (computed tomography) scanner, or of other tomographic modality.

During an imaging session, patient PAT may reside on a patient support PS (such as patient couch, bed, etc), but this is not necessary as the patient may also stand, squat or sit etc in an examination region ER during the imaging session. It is sufficient for the region of interest to so reside in the examination region ER/field-of-view FOV of imager IA. The examination region is formed by the portion of 3D space between the signal source SS and the detector device DD.

For example, in a CT setting, acquisition is multi-directional. For example, during imaging session, X-ray source SS rotates around the examination region with the patient in it to acquire projection imagery from different directions. The projection imagery is detected by the detector device DD, in this case an x-ray sensitive detector. The detector device DD may rotate opposite the examination region with the x-ray source SS, although such co-rotation is not necessarily required, such as in CT scanners of 4th or higher generation. The signal source SS, such an x-ray source (X-ray tube), is activated so that an x-ray beam XB issues forth from a focal spot in the tube during rotation.

The beam XB traverses the examination region and the patient tissue therein. and interacts with same to so cause modified radiation to be generated. The modified radiation is detected by the detector device DD as intensities. The detector DD device is coupled to acquisition circuitry such as DAQ to capture the projection imagery in digital for as digital imagery.

The same principles apply in (planar) radiography, only that there is no rotation of source SS during imaging. In such a radiographic setting, it is this projection imagery that may then be examined by the radiologist. In the tomographic/rotational setting, the muti-directional projection imagery is processed first by a reconstruction algorithm that transforms projection imagery from projection domain into sectional imagery in image domain. Image domain is located in the examination region ER. Projection imagery or reconstructed imagery will not be distinguished herein anymore, but will simply be referred to collectively as obtained imagery *m*.

The so obtainable imagery *m* may however not necessarily result from X-ray imaging. Other imaging modalities, such as emission imaging, as opposed to the previously mentioned transmission imaging modalities, are also envisaged herein such as SPECT or PET, etc. In addition, magnetic resonance imaging (MRI) is also envisaged herein in some embodiments.

For example, in MRI embodiments, the signal source SS is formed by radio frequency coils which may also function as detector device(s) DD, configured to receive, in receive mode, radio frequency response signals emitted by the patient residing in a magnetic field. Such response signals are generated in response to previous RF signals transmitted by the coils in transmit mode. There may dedicated transmit and receive coils however in some embodiments instead of the same coils being used in the said different modes.

In emission/nuclear imaging, the source SS is within the patient in the form of a previously administered radio tracer which emits radioactive radiation that interacts with patient tissue. This interaction results in gamma signals that are detected by detection device DD, in this case gamma cameras, arranged preferably in an annulus around the examination region where the patient resides during imaging.

Instead of, or in addition to the above-mentioned modalities, ultrasound (US) is also envisaged, with signal source and detector device DD being suitable acoustical US transducers.

The imagery *m* generated by whichever modality IA may be passed through a communication interface COM to a (non-volatile) memory MEM, where it may be stored for later review or other processing. The imagery may be rendered into a visualization by visualizer VIZ on a screen of a display device DID. However, an online setting is not excluded herein, where the imagery is consumed in whichever way as it is produced by the imaging apparatus IA.

Imagers IA of different modalities are envisaged herein, such as X-ray based (radiography, CT (computed tomography), C-arm, conebeam/dental CT, etc), MRI magnetic resonance imaging), nuclear imagers such as SPECT (single photon emission tomography)/PET (positron emission tomography), optical coherence tomography (OCT), ultrasound (US), etc.

In particular imagers of the tomography type (CT, MRI, OCT, nuclear), there is spatial domain between the signal source SS and the detection device DD that includes an examination region ER in which the patient, or at least part of the patient (the ROI) to be imaged, resides during imaging/data acquisition.

As said, and in more detail, the examination region ER is a portion of 3D space. Conceptually, the examination region ER can be thought of as made up of a spatial grid of spatial points (voxels). Such voxels help define tomographic sectional imagery. As will be explained in more detail below, whilst in some occasions the measurement data are of interest in their own right such as radiography, in some cases the measurements are further processed computationally to populate the grid of spatial points, voxels, with image values, to so build up a volumetric sectional image in the examination region. In this connection, the examination region is also sometimes referred to as the imaging domain, in distinction to the projection domain that is defined by a radiation sensitive surface made up of detector pixels of the detection device DD. Projection imagery (a visualization of the measurements $\lambda$ as such) on the other hand, of interest in radiography such as in "chest X-ray", or other, are spatially located in the said projection domain, and not in image domain.

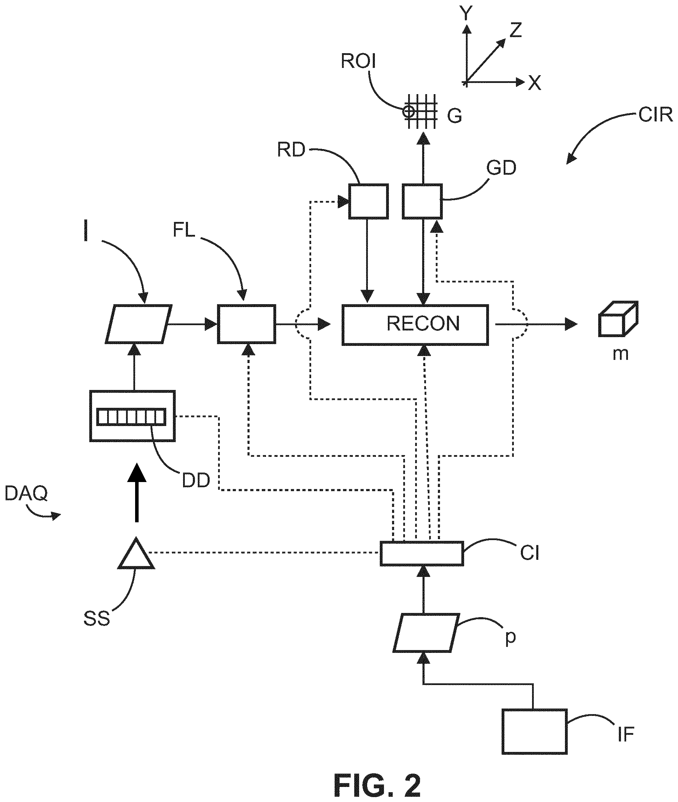

Overall operation of the imagery is through an operator console OC, a computing system with circuity such as processor (chip, CPU, etc), data memory/storage, I/O-interfacing, bus systems, etc, together operable by a medical user/staff (technician, doctor, radiologist, nurse, etc). Imaging apparatus IA is a complex entity. Adjustment of numerous machine settings of a multitude of components CIR (see Fig. 2 below of more details) of imager IA may be required, as dictated by medical imaging protocol and/or medical purpose/task for which the imaging is to be conducted.

Broadly, a given machine setting may be made up of one or more parameters that relate to settings of various such components CIR of the data acquisition section DAQ, or to settings of mechanical components that define the imaging geometry, or to any other component circuitry of the imager IA, etc. It is at least in parts the imaging geometry that determines the fields of view (FOV) of the imager IA. Imaging geometry may also include collimators, if any. Machine settings, as will be explained in more detail below, are one kind of imaging instructions, the automated provisioning of which is envisaged herein via the imaging facilitator IF in some embodiments.

Referring for now in more detail to the machine settings, such may be understood to define/control behavior, configuration, operational features, etc, of the various imaging components CIR that together make up the imaging apparatus IA. For example, components of data acquisition section DAQ generally relate to signal acquisition. Components in relation to imaging geometry relate to definition of the spatial configurations between the ROI (the part of the patient PAT to be imaged), and one or both of i) the signal source SS, and ii) the detector device DD. Imaging geometry defining components may include electro-mechanical machine parts, and related actuators AC that together act to realize different such imaging geometries by effecting motion (deformation, translation, rotation, or a combination of any two or more ore all of the foregoing) on such one or more machine parts with actuators acting thereon suitably. For example, such settings may relate to the opening (its size and/or shape) of the collimator, if any, thus forming the beam in X-ray based imaging. More generally, the imaging geometry may be adjusted by machine settings that control a spatial configuration of the source and/or the detector relative to ROI. For example, in X-ray based imaging, changing various angles $\alpha$ of source SS relative to a reference direction may determine propagation direction $\alpha$ of the x-ray beam through space towards the ROI. This in turn may determine the spatial information as encodable by the acquired projection data $\lambda$.

Examples of the above components may include: whole or parts of gantry GT, such as a movable gantry/arm component GT (in CT, or in therapeutic C-arm imagers, etc.). Gantry motions may allow angulations /tilts of source SS and/or detector DD. Other examples includes one or more motions in relation to table PS (if any), such as sliding or tilting thereof, relative linear translation through imager's bore, whilst gantry is rotating about longitudinal axis of patient on table, such as in helical scan, etc. On occasion, table PS remains stationary, but it is the gantry with the table PS (and thus patient) in its bore that is so translated past table.

However, higher generation of CT scanners are not excluded herein, where multi-directional data acquisition is not effected by mechanical rotation of source SS on gantry GT, but instead by switching multiple sources arranged in an annulus around the examination region/bore. In MRI, there are no such motions other than perhaps moving table with patient on it into bore. Instead, in MRI, multi-directional data acquisition is effected by switching into transmit/receive mode a set of RF coils arranged in different spatial directions. The coils are able to pick up measurements $\lambda$ as resonance signals, which signals may then be combined by MRI-reconstruction algorithms into sectional imagery/volumes of the ROI.

The above is merely a non-exhaustive illustration of operation of some components in imagers IA of various modalities. Other components in such or other imaging modalities are also envisaged herein, such as in nuclear imaging, US, etc.

In sum, each one of imaging geometry defining components and/or components of the data acquisition system DAQ can be adjusted by providing suitable machine settings. Ordinarily, this may need to be done manually by the informed user. Here however, it is proposed to assist the user to automatically, or at least semi-automatically, effect such machine settings, merely based on a natural language imaging request put through by the user, as will be explored herein in more detail.

Formulating such a request may not require detailed machine or even radiological knowledge. The request may merely take the form or an *"imaging wish",* a brief statement of what user wishes to accomplish imaging-wise, in addition to providing some basic facts of patient.

An imaging facilitator IF (to be described hereinunder in more detail) then translates this "wish" (imaging request) into corresponding correct machine settings for the imager IA at hand, and so readies the imager IA for the specified imaging task as per the request. The patient's (bio-) details may not even need to be provided explicitly. For example, patient ID information (name, patient number, etc) may be sufficient, as related patient bio-data may be pulled from a patient database, registry, HIS (hospital information system), etc, via suitable database interfacing, enabling information retrieval operations.

Triggering of the data acquisition (imaging) may also be done by the imaging facilitator IF, although in other embodiments this is left to the user. The imaging facilitator IF may indicate to the to user how such triggering is done, by a displaying a message, including textual and/or graphical elements, as preferred. In short, the proposed imaging facility IF greatly reduces the complexity of imaging.

Choosing the correct machine setting for each component is key for obtaining imagery of -- for the task sufficient -- image quality (*IQ*), such as of diagnostic quality. Choosing the correct such machine settings can be bewildering for the less experienced user, but even imaging pundits may struggle at times to get the parameters right when faced with workload stress and/or a new imaging apparatus, of different make and model, with which they may not be familiar with off the cuff. Trawling through pages on pages of a user manual may be time prohibitive in a busy radiological departments of large urban clinics/hospitals, *A&E* departments, trauma centers, or in field work (portable imaging by ambulance or Search & Rescue crews), or wherever needed.

Broadly, as proposed herein, the imaging set up includes the said imaging facilitator system IF. This may be integrated at least in parts into the operator console OC. In particular, a user interface UI of the imaging facilitator may be so integrated, but can also be supported by other computing entities as needed. The imaging facilitator IF allows a user, possibly inexperienced, to operate even a complex medical imaging apparatus IA according to protocol. It is important to note that some imaging sessions do not readily admit to repetitions in case the image quality of the acquired imagery turns up sub-par and undiagnostic on a first attempt. For example, some medical imaging protocols, such as X-ray based imaging, require dose exposure which can cause cancer. Thus, such exposure to toxic x-radiation ought to be limited to the extent absolutely necessary.

Liberal imaging repeats, such is in imaging with an optical camera until the "right shot" comes up, is in general prohibited in the medical setting. Thus, it is of benefit in medical imaging to get the machine setting *"right first time".* This can be daunting for medical staff less experienced in imaging, such a junior staff, or in places where medical education is hard to come by, such as in lower-income regions. But even in higher income regions, there is often the case that doctors are on circuit, such as locums, wo reside for short periods of time at different medical facilities. However, such different medical facilities may use imaging apparatuses IA of different make and/or models or modality, which the locum may not be familiar with. Thus, in these or similar circumstances, the proposed imaging facilitator IF may benefit such visiting medical staff by enabling them to efficiently and surehandedly operate even unfamiliar imaging apparatus, or when attempting an unfamiliar imaging task, etc.

Not operating imaging equipment correctly and thereby necessitating needlessly retakes, may not only come at the expense of health costs (eg, cancer due to X-ray exposure of patient and staff), but this may slow down imaging throughput, which is undesirable in a high-demand settings with lots of patients on waiting lists. In addition, unnecessary imaging retakes may place undue strain on imaging equipment, with consequentially premature wear and tear. This may attract unscheduled maintenance, downtime etc. In X-ray imaging for example, the anode disk in the X-ray tube SS is subject to considerable temperature gradients caused by the impacting beam of electrons within tube housing that are accelerated towards anode disk by the tube voltage, and that impact on the disk at the focal spot. Premature wear out of such disks may be a consequence of incorrect operation of such imagers. Such avoidable retakes may prematurely exhaust the planned imaging quota for the imager, with unscheduled service engineer callouts, downtime, repairs, etc.

In more detail, the imaging operation facilitator IF allows the user, such as the medical user, to set the imaging settings of imager IA. The imaging settings may be understood as one or more sets of imaging instructions. Imaging instructions pertain to information on how to conduct the imaging operation by imager IA, in order to achieve the imaging purpose/task, with sufficient IQ. The imaging instructions that were mentioned above so far are machine settings and pertain to the functioning of the imager, its components, etc. However, there may also be a different set of imaging instructions of a different kind, directed to the patient. Thus, as envisaged herein, imaging instructions come in at least two flavors: i) the machine-sided ones (the mentioned machine settings), and ii) patient-sided instructions, that pertain primarily to patient conduct, prior or during imaging (data acquisitions). Machine-sided instructions may be denoted herein as $p_m$, whilst patient-sided instructions may be denoted herein as $p_p$. Image instructions in general may be denoted herein as $p = (p_m, p_p)$ or simply *as p*.

Imaging instructions $p$, in particular of the machine-sided kind, may be understood as machine settings as mentioned above. They may be represented as a set of parameters, such as numbers. The parameters may be represented in list form. Some or more of the parameters may be of matrix or vector form. All parameters may be of thought to be consolidated into a super data structure, such as (super)matrix, a tensor, an associate array, or in any other form. The patient-sided settings may be understood as motion or, more generally, behavior requesting instructions. They too may be represented by numbers, vector, matrices, etc. All imaging instructions, patient- and machine-sided, may be thought to be represented in one single encompassing data structure, such as a tensor of suitably large dimension. Alternatively, the machine settings may be presented as a sequence of symbols, data structures, numbers, etc,

As to the patients-sided instructions, they may relate to a pose or behavior correction instruction. "Pose" as used herein, relates either one or both of patent/ROOI position and/or its spatial orientation, relative to source SS and/or detector DD. The patient-sided instructions may be instruction on which pose the patient is assume and/or which behavior they ought to exhibit before and/or during the imaging.. For example, the patient is supposed to conduct a breath-hold before or during an MRI or CT scan. In addition, or instead, the patient may be required to assume a certain pose, such as a specific orientation and/or position of one or more body parts in relation to in particular the image acquisition components, such as the source SS and/or the detector DD. For example, in a chest x-ray, patient is sometimes standing in the examination room and he or she is assumed to stand in a certain way, to breathe or not, etc, when exposing the chest to the X-ray tube SS with the detector DD behind. Patient-sided instructions, together with the machine-sided instructions for imaging geometry components, may together define the correct imaging geometry to be realized for best image quality (IQ).

Broadly, the imaging facilitator IF processes the imaging request, and outputs., based on such processing, a data structure in the requisite (eg, pre-agreed) data dimension/format to so represent the wanted imaging instructions p. Alternatively, if the imaging request is in free text form, so may be the output, a text of agreed length that conveys to user how to implement the image instructions. The imaging facilitator IF may be configured to output machine-sided instructions $p_m$, or patient-sided instructions $p_p$, or both, as needed.

Thus, for either one of both of said imaging instruction flavors, the machine-sided and patient-sided imaging instructions, the proposed imaging facilitator IF is operable to provide such instructions in an automated manner, upon provisioning by user of imaging request input data. The request data may be purely "phenomenological", and do not require the specification of any imaging instruction parameters. In fact, the user may not be cognizant on such parameters at all. This may greatly ease the burden on user, and allows even inexperienced users to safely and efficiently operate medical imaging equipment, and/or allows experienced user to do so with yet more efficiency, increasing overall imaging throughput.

Before explaining the operation of the facilitator IF in more detail, reference is now made first to Fig. 2 which shows the manifold components CIR that need to be provided with imaging instructions $p_m$.

Imaging facilitator IF provides such, preferably machine-sided instructions, $p_m$. These are propagated through control interface circuitry CI, including suitable interfacing API's, to the various components CIR of the medical imaging apparatus IA.

The imaging controlling components CIR of imager IA may include the signal source SS, the detector unit DD. More generally, such components may be members of the data acquisition unit DAQ operable to acquire the measurements $\lambda$. In addition, downstream to acquisition, the acquired measurements may be processed, such as by a reconstruction algorithm RECON, to compute tomographic or volumetric imagery $m$ in image domain. However, in solely projection-based imaging such as radiography, output image may be still in projection domain. Thus, in some embodiments, the output imagery $m$ is, or includes, the measurement data $\lambda$.

The settings may also relate to certain parameters of a filter component FL, or indeed to parameters such as regularizer parameters, or other parameters of the reconstruction algorithm RECON or filter component FL to be used.

For example, such tomographic reconstruction algorithms RECON may include filtered back-projection (FBP), iterative, algebraic, or machine learning based as needed.

The region of interest definer RD relates to an imaging geometry defining component. As mentioned, such may effect mechanical movement of the gantry for example, and thus of the source SS and/or the detector DD, and/or may effect motion control of the patient support PS, such as in CT or magnetic resonance imaging. Another imaging geometry defining component may include the collimator in X-ray. Thus, some parameter(s) $p_m$ may relate to positions of radiopaque collimator blades of collimator.

The grid definer GD defines the mentioned spacing of the spatial grid and voxels in a coordinate system image of the examination region $(X, Y, Z)$. The resolution for example may be set in this manner, based on a suitable parameter of the imaging instruction.

In addition, the imaging instruction $p_m$ may also include parameters for the visualizer VIZ, such as to set level and windowing parameters to optimize the manner of displaying the image values. Level/windowing describes the mapping of image values in imagery m to a range (a "palette") of color or grey values. For example, CT imagery m may have a vast dynamic image value range, and displaying such imagery at wrong settings may drown out details that may be crucial for the imaging purpose at hand.

With continued, and with yet more detailed, reference to Fig. 2, this is a schematic representation of a (tomographic) reconstruction pipeline RP as may be used herein, for CT., or MRI, or other. Thus, the block diagram of the reconstruction pipeline RP illustrates various components, some of which may be optional. Some or each of the components may be controlled by respective parameters p, which together form the imaging parameter (setting) $p_m$.

The projection data $\lambda$ as measured at detector DD along different projection directions $\alpha^d$ is receivable at a projection data input port (not shown) of the pipeline PR. The received projection data is optionally processed by a filter component FL. The filter component FL may be operable as a band pass filter, a low pass filter or a high pass filter, or any other. The filter component FL may be operable as a noise filter to reduce noise in the measured projection data $\lambda$. A noise model may be used such as statistical or any other.

The optionally filtered projection data is processed by a reconstruction component RECON into reconstructed imagery in image domain G. The reconstruction component RECON may implement a reconstruction algorithm.

A reconstructor component RECON may implement one or more reconstruction algorithms to process the projection imagery into imagery in image domain, such as the preview imagery or the final image. Specifically, the reconstructor RECON may compute the output image m as sectional imagery of the examination region (with the patient in it) for diagnostic, therapeutic or other purposes. The reconstructor RECON may be able to produce sectional volumetric image data m. However, this does not exclude producing a single image slice in the examination region as required. Thus, the reconstructed imagery may include the whole volume ER, a partial volume thereof, or a specific section therethrough. Volumetric reconstruction may be facilitated by helical movement, and/or the 2D layout, of the X-ray detector DD.

Any suitable reconstruction algorithm may be used by reconstructor RECON component, such as algebraic, statistical or analytical. Analytical reconstruction algorithm may include filtered back-projection (FBP). Iterative reconstruction algorithms are also envisaged in preferred embodiments.

For example, tomographic reconstruction algorithms as envisaged herein can be formulated as an iterative procedure for improving an objective function. Specifically, the procedure may be formulated in terms of optimization, in particular minimization, of a reconstruction cost function. The reconstruction cost function measures how well the estimated imagery in image domain provided during the iterations matches the actually observed projection measurements $\lambda$. A system matrix may be used to forward-project the reconstructed imagery into the projection domain and to compare this forward projection with the actually measured projection data $\lambda$. A deviation between the actually measured data $\lambda$ and the forward projection as per the system matrix can be quantified by the cost function. In the iterations the imagery in image domain is adapted to reduce the reconstruction cost function $q$, the algorithm thus converging towards the correct imagery. A dual formulation of the above described optimization is also contemplated which includes a maximization of a utility function.

In more detail, an iterative tomographic imaging reconstruction algorithm can be described as follows (excluding any attenuation correction for simplicity):

$$\lambda_e = FP^\alpha[\mathrm{f}] \tag{1}$$

$$f_i^{\,j} = BP^\beta \left( \lambda_e, \lambda m \right) \tag{2}$$

$$f_{i+1}^{\,j} = U^\gamma \left( f_i^{\,j} \right) \tag{3}$$

wherein:

$\lambda$e is estimated projection data, such as in list or frame/sinogram mode/format;

$\lambda$m is projection data measured at detector device DD;

*FP* is a forward projection operator and may be implemented as a matrix, sometimes referred to as a system matrix, that describes a forward projection from image domain into projection domain;

*BP* is a backward projection operator that estimates voxel values in grid G from projections;

$f^i$ is the *i*-th voxel in the imagery in image domain to be reconstructed;

U is an reconstruction update function that updates voxels *f* during the iterations *i*.

The nature of reconstruction update function *U* depends on the optimization algorithm used and is implemented by an updater module (not shown). Embodiments of updater function U may include update functions based on any one or more of the following numerical methods or techniques: gradient descent, stochastic gradient method, conjugate gradient, Nelder-Mead expectation maximization (EM), maximum likelihood methods, or any other. The goal in the said optimization is to adjust the voxel values to that the residue as per reconstruction update function cost function $q = \| \lambda_m - FP[f^i] \|$ becomes sufficiently small, for example drops under a pre-defined threshold. $\|.\|$ is a suitable similarity measure, such as Euclidean, least squares, or some suitable $L_p$ norm.

In eqs (1)-(3) above, the operators expressed there are a function of certain parameters $\alpha$, $\beta$, $\lambda$ that respectively control the behavior of the respective operator as per eqs (1)-(3), and thus of the reconstruction operation as a whole.

Any of the above mentioned components, such as the filter component FL, the ROI definer RD, the grid definer GD and the reconstruction component RECON may be arranged as separate, discrete, components as shown in Fig. 2. However, this may not necessarily be the case in all embodiments, as the above, "spread-out", layout in Fig. 2 is largely driven by clarity of presentation. Thus, in other embodiments, some or all of the components may be coalesced into fewer components by integration or combination, or even into one single component. For example, the filter element component FL is sometimes part of the reconstruction algorithm implemented by the reconstruction component RECON.

Operation of each or some of the said pipeline PR components may be a function of the said imaging setting parameters $p_m$.

The facilitator system IF may a control interface CI through which different such machine settings $p_m$ can be provided, and fed into the reconstruction pipeline RP. Thus, the facilitator IF allow controlling operational behavior of the pipeline. Thus, the interface CI populates memory placeholder (free parameters) related to the various components, with the received parameters $p_m$, and so sets them to certain values, thus controlling operational behavior of the pipeline PR.

Such parameters that make up the machine setting may include parameters of the noise model and/or of the reconstruction algorithm, such as any one of $\alpha, \beta, \lambda$ in eqs (1)-(3) above, and others still,.

Yet other parameters $p_m$ of machine setting may include one or more parameters of the noise model, one or more parameters of the reconstruction pipeline, in particular, of the reconstructor RECON in itself such as certain parameters any one of parameters $\alpha, \beta, \gamma$ (see eqs (1-3) above) of the reconstruction algorithm, and as said, the size of the grid, the number of projection data frames to be used, the size of the ROI to be reconstructed in image domain, the number of iteration cycles, etc. Other parameters for the parameter setting may include regularization parameters, strength modulators such as me be configured in a denoising /noise suppression algorithm of the filter FL, or in the iterative reconstruction algorithm used by reconstructor RECON. Further parameters may include shape parameters of the reconstruction cost/utility function q as may be used in the iterative reconstruction algorithm by reconstructor RECON Such parameter(s) control behavior of reconstruction updater function *U*, etc. Another type of parameters in the settings P may set the type of algorithm, for filtering and/or reconstruction, to be used.

In MRI, the parameters $p_m$ may include pulse sequence definitions, that is, a list of instructions of which of the RF coils $C_j$ ($C_x C_y$, $C_z$) to energize, by how much, for how long, and when, in which order, etc. Other parameters may relate to MRI reconstruction implemented by reconstructor RECON, such as operations in *k*-space, Fourier filter coefficients, manner of read out from *k*-space, and may others.

The above list of parameters are merely illustrative and non-exhaustive.

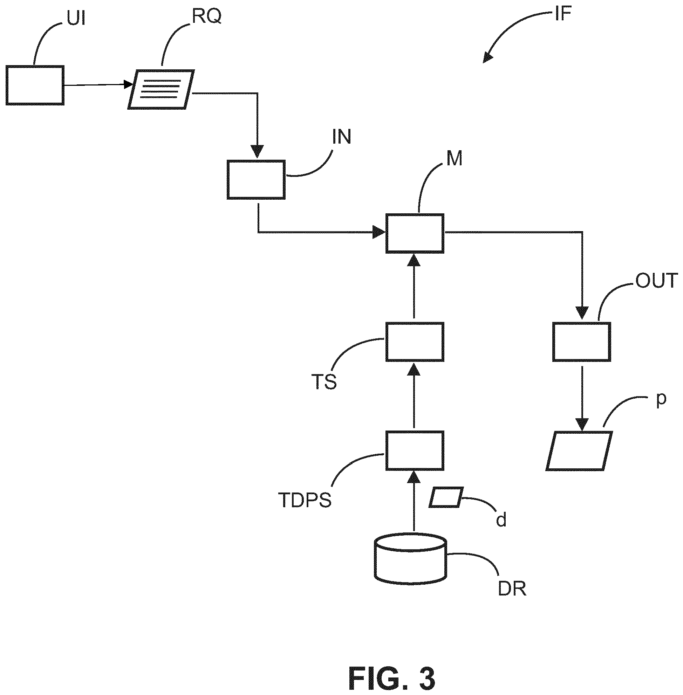

Reference is now made to Fig. 3 which shows in more detail the imaging facilitator IF. The facilitator IF may include one or more input ports/interfaces IN through which input data is provided by user to the facilitator IF. Such input data may include the said imaging request RQ, which can be in free text form, or in other format. The imaging request may describe in descriptive terms, the imaging objective to be achieved. The facilitator IF may be machine learning based. The facilitator IF may include a (that is, one or more) trained machine learning models M. Thus, imaging request input data QR is fed into the trained machine learning model M. The machine learning model M processes the input data to produce, based thereon, output data. The output data may be output as the said image instruction p at output port OUT.

For example, the patient-sided imaging instructions $p_p$ may be output as a text for the attention of patient PAT, who can read these off a screen of a display device mounted in the exam region, e.g., integrated into imager's bore etc. Alternatively, or in addition, such are displayed to user and he/she reads those off and explains patient what they should do, how to move, perform a breath-hold, and when, etc. Existing automated speech generation models can also be used to generate artificial speech from the text, in a language, dialect, accent, etc, or mimicking language style of a patient selected person (favorite movie actor, etc), as preferred by user. Such may have a calming effect on the patent and may foster better compliance. The output patient-sided imaging instructions may be used to generate a visualization by visualizer VIZ (shown in Fig. 1), such as instruction text, as still image or video rendering, a graphical illustration, etc, on screen of display device, for user or patient. Alternatively, or in addition, video sequence, footage, etc, is caused to be displayed on screen of a patient accessible display device, either generated by a generative model or selected from a connected database of video/image material. Instead of, or in addition, such footage may be displayed to user.

A non-ionizing optical camera SEN (shown in Fig. 1), such as a still or video camera, capable of optical imaging, depth sensing, IR, NIR, LIDAR etc, may be used to capture a current position/orientation of patient/ROI, and this is compared with a known ideal pose. If there is deviation, the patient-sided instruction $p_p$ is issued as remedial motion, acts, which the patient should be exhibiting to ensure IQ by assuming the correct pose, or by performing requisite acts (breath-hold, etc). The non-ionizing camera SEN may be arranged in the exam room with a suitable field of view (FOV) so that imagery of at least of the relevant ROI of patient can be taken. Alternatively, or in addition, the patient monitoring camera SEN is integrated into imager IA, such as in or at its bore, gantry, etc.

As to the output data including machine-sided imaging instructions $p_m$, such may also be displayed to user on a screen of display device DID, or may be sounded out, as needed. For example, the machine-sided imaging instructions $p_m$ may be displayed in free text form, or in tabular form, in coded form, such as in a table, matrix, vector, etc, which lists the parameters vs the respective component to which they pertain, etc. The output data may in addition include information on how the machine settings are to be applied to the imager IA, where/how these are to be input, etc. An explanatory summary may be provided that not only mentions the specific machine settings (eg, in numbers, values, such for example *"tube voltage = 60kV",* etc) but also explains how these are to be applied /effected, which GUI menu of the imager's user interface/console OC to go to, which buttons on the operator console OC to press, when, in which order, etc. Alternatively, or in addition to indicating the output machine settings to user, the machine settings may be applied via suitable control interfacing/circuitry, APIs, etc, to the relevant component interfaces automatically, as mentioned above in connection with Fig. 2. Microcontroller(s) may be operated in this manner that effect the machine settings. Controller Area Network (CAN bus) setups, or other machine control infrastructure, may be used for effecting machine settings, etc.

As mentioned, the imaging request RQ may be provided by user in free text form, such as an ASCI file, via an editor functionality for example, included in user interface UI. The imaging request RQ can be formulated as a question by the user. For example, through a suitable user interface such as a graphical interface with a text field, user may provide the imaging request as a text by inputting: '*I wish to image a 65 year old patient, male, for a suspected heart problem by using a Philips SQP imager, model SKP2, model year 2016'* (the model, make etc, are completely fictional, and are used herein purely for illustration purposes). Input may be by keyboard input, touchscreen gestures, pointer/click tool, or in any other form.

Thus, the proposed imaging set up supports automated image instruction generation. The user in the imaging request, can even supply the specific make and/or model (even model year) of the imager which is to be used for the imaging session. As said, text input can be via touchscreen action, stylus, digital pen, or keyboard, etc. Input via speech may also be envisaged, using suitable data capture and conversion circuitry, such as NLP pipeline in communication with transducer (microphone, etc) and A/D circuitry, as needed. User input via free-text or speech has been found to allow operating the imager IA by users with different sophistication levels. For more sophisticated users, with more in-depth prior knowledge, a more structured input interface may be envisaged, such as GUI menu-guided, etc.

Such quick and surehanded imaging thanks to the imaging facilitator IF allows achieving a higher patient throughput, with fewer medical professionals handling a large volume of patients which is preferrable in particular in times of cash-strapped health systems with a smaller medical workforce facing an aging population.

In whichever form the imaging request RQ is provided, it may include, in particular, a specification of patient data (such as bio-characteristics, such as gender, BMI, etc) and the imaging task that is to be accomplished. The designation of make and model is preferred, but is optional. As mentioned earlier, it may not be necessary for user to explicitly provide this information as input. A patient ID may be sufficient, and such bio-data and/or imaging task may be pulled from patient's health records. Also, the model and make of imager may be implicitly set.

In order to be able to provide such imaging instructions that is imager model and/or make cognizant, the facilitator IF may have access to a bank of machine learning models M= $(M_1, M_2, ... M_j, ... , M_n)$, each ML model $M_j$ trained or fine-tuned for each specific imager make/model. In this case, the user may first select the imager IA make/model. The facilitator system IF then accesses the associated ML model and performs the computations through it to obtain the applicable imaging instruction. Then, the imaging request RQ is fed to the associated model to produce the output $p$. Alternatively, a single ML model is trained that is capable of resolving imaging instructions p down to imager model/make, or even model year, as needed.

The imaging facilitator IF may include, of may be able to communicate with, the said user interface UI through which the imaging request *RQ* is provided. The imaging facilitator IF may be fully or partly integrated into the operator console OC of the imaging apparatus. Some or all functionalities may be carried out by other computing system, possibly arranged remotely from imager.

In particular, the execution of the machine learning model for processing the input imaging request may consume considerable storage and/or CPU compute power which may not be readily available on site, where the imager itself is located. In such settings, the machine learning model may be stored and or executed in one or more servers in the cloud or on edge computing device(s). Preferably, the computing entities may use chip sets of multi-core design, capable of parallelized computing. Distributive computing may be used to balance the workload when executing the model. Such distributed computing setups, with parallelized arrangements, may also be used, or may be used instead, when training the model, as training may consume even more storage/compute resources than does deployment (when using the trained model in clinical practice). On the other hand, the user interface functionality through which user provides the imaging request RQ, and the data telecommunication management, such as sending request RQ to model M/receiving instructions p from model, may be run form a more modest client end device. For example, imaging request is composed in an editor on the client end device by user, is then sent through a communication network (not shown) to the one or more servers that are orchestrated to perform the machine learning based model operations on the input data to produce the output p. Once this is produced, output p is sent down through the communication network back to the user device. It may then be stored in a buffer and/or is indicated such as displayed, sounded out, at user's end device. End device may be any computing device, such as a smart phone, laptop, desktop, tablet etc.

Training & Models

The imaging facilitator IF may include memory, storage, memory/storage control circuity, processor circuitry (microprocessor, chip), I/O circuity, etc, to effect executing the trained machine learning model M.

The machine learning model M, prior to deployment, was trained by a computerized training system TS in one or more training cycles. Training is based on training data d, obtained from a data repository DR such as historical imaging data, patient records etc. Such historical data may be augmented to induce better variation to make training more robust and to avoid overfitting.

In general, the training data includes, in whichever form, patient data, and imaging instructions that were applied in historical imaging sessions, to obtain historical imagery. In addition or instead, ML based synthesizing of training data is also envisaged herein, if sufficient quantities or sufficiently varied historical training data is unobtainable, or require prohibitive overhead. Such data synthesizing may itself be ML based, such as by using suitable generative setups (eg, GANs, mentioned in more detail below).

Data training provider system TDPS provides the training data d to the training system TS for processing, that is, for training the model. Training may be done in one or more pre-training cycles for example, and then (using other training data) in one or more subsequent training cycles (fine-tuning), based on training results of the pre-training.

Broadly, the training system TS runs a machine learning algorithm that adjusts parameters of the machine learning model. Performance of the model can then be checked by running a test session. Once sufficiently low error rate is achieved on test data, the model is considered trained and can be used in the imaging facilitator IF, or ca be passed on for fine-tuning, etc. Thus, model M can be released for deployment in clinical practice where it is exposed to new input data it has not necessarily "seen" before, to produce output data, such as the imaging instructions $p$, which allow an error reduced, patient tailored, less cumbersome, more automated, and thus smooth imaging experience for staff and patient.

The training arrangement including the training system TS, and optionally, the training data provider system TDPS are shown in Fig. 3 merely for completeness. They do not necessarily form part of the medical imaging arrangement, at least not during deployment periods. Thus, once the model is trained, the training system TS, training data provider TDPS etc, is no longer needed during operation, until the next training cycle, if any.

The training may be one-off, but, preferably, can be repeated as more training data in a suitable variation emerges. For example, and in more detail, multiple training cycles may be used, with pre-training on more general data, with subsequent one or more training cycles on more specialist training data. The training data may be in text form.

The training data provider (system) TDPS may be arranged as a web-crawler, and/or a database information retrieval component that formulates suitable queries for obtaining training data $d$, etc, from existing imaging data repositories..

The training data provider (system) TDPS may preprocess the training data such as by "masking" to artificially blank out parts of the text in a sentence or paragraph(s), etc of training data, to so obtain training input $x$ (the text portion that remains after masking), and its associated target $y$ (the masked text), $d = \{(x,y)\}$. In this manner, self-supervised training may be implemented. The so produced training data can then be passed on to training system TS for training, Other self-supervised training setups are also envisaged, and so are supervised training, un-supervised training, re-enforced training, etc.

In an exemplary supervised setting, historical image data may be localized in a database query by training data provider (system) TDPS across medical image repositories, such as in PACS, etc. Imagery of sufficient IQ may be identified using entries in the image repository. If the image was of insufficient IQ, this fact may be indicated, and such imagery may be ignored herein, or such IQ-deficient imagery is simply not loaded in PACS and cannot thus undermine the search. Thus, an assumption can be made that all imagery in a PACS, or other "official" medical image repository of a medical facility, are by definition of sufficient IQ. Patient's data to which such imagery pertains may be stored as training input data $x$. Such training input $x$ may be sourced from patient records, that may be linked to PACS imagery. If the imagery is in certain formats, such as of DICOM or similar, metadata, such information x may be found in image header data, can be grabbed and stored as target data y, associated with input x. Such metadata may include the, historical, machine settings $p_m$ that were used back then to obtain the respective historical imagery. Looping over such historical imagery for the same patient and different patients, and storing related instants of $(x,y)$ allows building up a training data set $d$.

Training for patient-sided instructions may be also so sourced from historical data in some cases, or may be synthesized. For example, video footage/or still imagery (as acquired by a camera, such as the camera SEN mentioned earlier) of patient poses are shown to (human) medical imaging experts, who provide pose correction information appliable to the instants shown. The pose corrections (if any) or confirmatory feedbacks are recorded and associated with the image material on the patient poses to so obtain training data samples. The patient poses may be acted out by actors/volunteers, or are recorded in real clinical practice over a training data gathering period until a sufficient supply of "ground truth" training data is build up. Optionally, GAN-type or other generative ML models may be used to synthesize even more data from this ground truth supply.

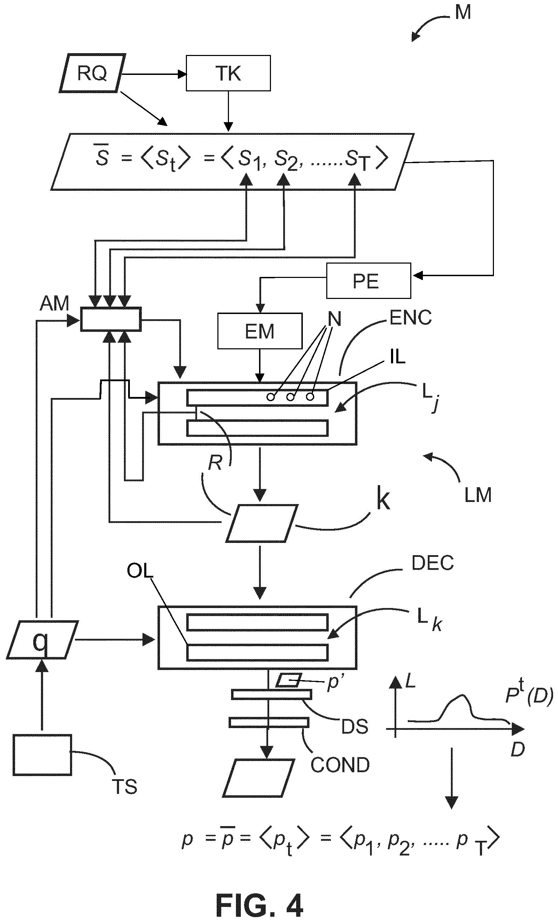

Reference is now made to Fig. 4 which shows more details of the machine learning model M as may be used herein in embodiments for the imaging facilitator IF.

Preferably, the machine learning model is of the generative type. Generative type machine learning models are trained to sample from a probability distribution $P$ of a population of interest such as over a dictionary of tokens (language fragments), or more general, from a high-dimensional data set ("landscape") of imaging instruction parameters.

In some such embodiments, but not necessarily in all such embodiments, the machine learning model is envisaged herein as one of a natural language model, thus is capable of natural language processing. Such is envisaged herein in particular in the above mentioned *"text-2-text"* embodiment, in which the input imaging requests RQ is received at the model in (free) text form, and is processed by model into the output imaging instructions p, preferably also in text form. For example, the model may act as a summarizer, to convey to user in a summary text (of pre-set length) what user needs to do for accomplishing the imaging task for given patient, which parameters to set, and how, etc. However, variations of this text-based format are also envisaged, where input and/or output is more structured, possible coded, in a table, etc, using symbology other than, or in addition to, text, in various formats.

More generally, the model M may be of the generative type and configured for sequence2sequence data-type processing. The sequences involved may or may be natural language text, depending on the use case.

The imaging request RQ may also include additional data, in particular patient contextual data, such as prior imagery of patient, parts or all of patient's health record, etc. Such patient contextual data may serve as useful data-driven regularization that may help the model to build better correlations, deliver more robust results, etc. Such contextual data may be preferably used only during training, but may be used instead, or in addition, in deployment/testing.

The top of Fig. 4 shows a schematic rendition of the input imaging request RQ, as a string of data $s = \overline{s}$. The data may be presented as a sequence of symbols such as word fragments (tokens, eg N-grams, N>=2, or concatenations $c(s)$ thereof) of word fragments, of whole words, of phrases of words, sentence(s), paragraphs. etc, of natural language, or in coded form, as provided by the user. Examples of such coded form for the output imaging instruction p may be a vector or matrix (table). For example, some or each position may correspond to a component, or part of component, of imager IA, and its value to a corresponding parameter setting, such as $p_t$ = *"60kV"*, it being understood that the given position $t$ in sequence p represents the tube voltage in X-ray imaging for example. Other positions may relate to other components, such as collimator, etc.

More generally still, and as mentioned above, the model M is configured herein in some embodiments for sequence-2-sequence processing where an input sequence of elements $(s_j) = s = \overline{s}$ (the imaging request) is transformed into the imaging instruction $p = (pt)$, likewise different sequence of elements, which may or may not be a natural language statement. Elements or "tokens" will be used herein as a general reference to any part of the sequence. Thus, each token $s_j, p_t$ may include individual words, fragments thereof (such as one or more letters), more generally referred to as tokens, such as an N-gram $(N>1)$, such as a 2-gram, or other. A similar convention will be adopted herein for the input sequence s. Some token may code for word separators, such as "white space", that is, empty/separating space between two non-empty tokens. Whilst the tokens herein are mainly conceptualized for textual fragments of natural or coded language, they may instead related to speech/sound fragments, in acoustics, such a phonemes, or parts thereof. Thus, the modelling envisaged herein may be capable of natively processing speech.

In such tokenized, sequential data-based embodiments, tokenizer TK breaks down the input sequence s (the imaging request RQ) into a list of the tokens $(s_j)$ arranged in a particular order as schematically indicated by index $j$. Each such token thus has a certain in-sequence-position, the position being indicated by the index $j$. Similarly, for the input output sequence $(p_t)$, with index $t$. The positional information may be also encoded otherwise than by ordinal indexing, such as by mapping to an aspect/parameter of trigonometric base function(s). Whichever way, the positional encoding is preferably done in a manner such that it can be maintained and tracked throughout the processing of input into intermediate data R, and the processing of such intermediate data R into output data. A positional encoder PE may thus be used to effect such positional encoding. The sequences $\bar{s}$, $\bar{p}$ may have different lengths and may have completely different composition as is expected. However, the in- and output length is preferably pre-set to reduce processing time.

In some embodiments, the machine learning model does not directly operate on the input sequence $s,p$ of tokens, but instead an embedder *EM,* that operates on tokens s,p to map into embeddings *EM ->$f_s(s)$, $f_p(p)$.* Thus, embedder may be used to convert sequence into a corresponding vector/point $f_s(s)$, $f_p(p)$ in an embedding space, or "meaning space". Such space may have the structure of an (affine) vector space. The model then operates on the embeddings $f_s(s)$ of the input sequence *s*, instead of on the sequence s as such. The vector $f_s(s)$, $M(f(s))=f_p(p)$ may be high dimensional, and may present as a ordered sequence of numbers. As indicated by the subscripted notation, the embedding operations $f_s$, $f_p$ at in- and output may be different, although using the same embeddings is not excluded. However, in the following we will, for brevity, simple refer to "the embedding", as a shorthand for either at in- or output, or, if the same, for both. The embedding is so chosen that a token, or group of tokens, with similar meaning are mapped to vectors/points in closer proximity. In that sense the vector space can be understood to be a "meaning space", with points/vectors therein indicating concepts, and their points/vectors in closer proximity representing similar or related concepts, words, phrases etc. Thus, the embedding vector space may have a distance function, such as Euclidean distance, defined thereon to quantify such proximity. The embedding is such that it is reversible, so that the output embedding $f_p(p)$ can be reversed into the output sequence *p.* in the following we will drop the notion *fs(s), $f_p(p)$* etc, to disencumber notation, it being understood that a reference to data sequences RQ, *s,p* may include a reference to their respective embeddings $f_s(s)$, $f_p(p)$, with the understanding that one can be readily converted into the other.

Any one of tokenizer TK, positional encoder PE and embedder EM may not necessarily be part of the model M proper, but may be formed as respective pre- and/or post-processing pipeline(s), to which model M may be interfaced.

Broadly, the machine learning model M may be arranged as a cascaded sequence of layers L= ($L_j$, $L_k$).

There may be an input layer IL at which the input s is received, and the output layer at which the output *p* is produced. There may be plural hidden layers in between out- and input layer, where intermediate data is passed from input layer to hidden layer, and from hidden layer to hidden layer, to so yield, in the end, output p at output layer OL. Multiple such hidden layers may be said to implement a "deep" architecture, with the number of hidden layers determining the depth of the architecture.

Each given layer *L* may comprise multiple computational nodes N, that apply a respective computational operation, such as linear combination, or any other functional operation, to any incoming input from node(s) of the previous layer to produce output which is then passed on to the next input for the next layer and so on in a cascaded manner. The input layer IL may so process the initial input sequence s.

Same layers may implement a non-linear function (activation function) that is applied to the output produced by one or more nodes of a previous layer. It is the response value of this non-linear function that is then being passed as intermediate data R to one or more nodes of the next layer, and so forth. Such non-linear activation functions may include any one of soft-max function, *arctan* function, sigmoid, *tanh,* and *ReLU* (Rectified Linear Unit), or others still. Preferably, the non-linear function may be continuous, so the training may be (better) handled by gradient-based methods.

Some layers implement different operations, such as an attention mechanism AM as shown to the left of Fig. 4. Attention mechanisms AM are computational arrangements that score, as soft parameters, relationships or contexts between tokens, either in any given sequence (self-attention) which can be the input sequence, or an intermediate data sequence produced as output by a hidden layer, etc. Alternatively, the inter-token relationship is so modeled between sequence in- and/or output from different layers, that may be multiple layers apart. Thus, the attention mechanism AM can also be used to establish relations or relation scores between the tokens of input s and the tokens of output *p*. Such soft parameters that score the pairs or n-wise (n>2) relationship between tokens from part of the set of model parameters θ that are adjusted during training. Attention mechanism AM may be configured for self-attention, which allows expressing relationships for tokens within a given sequence, either at input layer or in intermediate data R. Attention mechanism in general may operate to code for (express) such relationships for tokens from different sequences. General attention, and self-attention may be used in combination herein. Attention mechanism allows more efficient training and processing, as compared to sequential processing previously needed in recurrent networks RNN, or and long-short-term-memory (LSTM) setups, or others. Thus, whilst the data itself may be sequential, the internal processing may not need to be so restricted, but may instead be parallelized. Also, large amounts of data can be more efficiently trained and processed, based on parallelized processing, preferably using multi-core chipset hardware, either generic or dedicated, such as GPUs ("graphical processing unit"). Whilst the processing herein does not necessarily relate to graphics processing, it has been found that the processing in deployment and training can be represented by vector or matrix operations such as dot-products (Attention mechanisms) or matrix multiplication. For example, in attention mechanisms, dot-products may be used to measure how nay two tokens are related to each other. It is such operations that can be handled efficiently by such graphics hardware (the said GPUS, etc), with more responsive return of results p in deployment and/or shorter training times. More than one attention score may be computed per pair of *n>2* tuple of tokens, in particular in a multi-headed attention mechanism, also envisaged herein in embodiments. Attention mechanism as such have been previously described. For example, by A. Vaswani at al, in "Attention is all you Need", published in the arXiv repository under reference code arXiv:1706.03762 [cs.CL] (2017), available online https://arxiv.org/abs/1706.03762.

The machine learning model may be based on pre-trained large language model (LLM) architectures, such as previously proposed. For example, on such model ("BERT") is reported by J Devlin et al in "BERT: Pre-training of Deep Bidirectional Transformers for Language Understanding, reported on the arXive platform, under code arXiv: 1810.04805v2[cs.CL] (2018) or the "GPT" models in its various generations, such as GPT-2, -3, -4 , as described for example in "Language Models are Few-Shot Learners", by Brown et al, published on arXive, under code ar-Xiv:2005.14165 [cs.CL] (2020), available online at https://arxiv.org/abs/2005.14165.

Broadly, the model may be one of the generative type, with autoregressive processing, preferably any one or more of pretrained and with attention mechanism. Some such models any have an encoder-decoder architecture, as will be explained in more detail below. In more specific embodiments, the overall architecture may be that of a *"transformer",* a pre-trained model seq2seq model, with attention mechanism, similar as previously described for instance in the cited paper by *Vaswani et al.*

Instead of using such encoder-decoder paradigm, some language models envisaged herein may be of the encoder-only type, or of the decoder-only type, as preferred. Having said that, nothing herein is necessarily tied to any of the above, in in particular to the said LLM type models. Instead, any one or more of GANs, variational autoencoders VAE, diffusion models, etc, may be used instead or in addition in various combinations. Any other suitable alternative modelling, not necessarily of the NLP type with free text processing, may be used instead, such as decision trees, forests thereof, etc.

In general, the output layer OL may function to process intermediate output R that it receives into the desired format of the output imaging instruction *p*. Such format may be the desired text length of the imagining instruction text, or the size of the table, such as vector, matrix, or tensor, or whatever the desired format, structured or not.

The input/output and/or the intermediate data may be itself be represented suitably as vectors, matrices, or tensors (higher than two dimensional matrices with more than two indices to indicate each element therein).

In embodiments, at least a part of the machine learning model is arranged as an artificial neural network (NN). In some such embodiments, at least some of it is either a fully convolutional or a fully connected network, as needed. In convolutional networks, some layers represent convolutional operates comprising a group of filter coefficients. There are applied to (true) subsets of intermediate output from previous layers, one at a time, for each output of such convolutional layer. Thus, each output element is function of a true subset of all input received from previous layer. This may be in contrast to fully connected layers, where each output is a function of all input elements received from the previous layer. In some embodiments, such as in those with attention mechanisms AM, no convolutional layers are used. Unlike convolutional processing, where the group of filters remain constant for the processing of any feature map/intermediate data in a given layer, in attention mechanism, the inter-token relational context scoring may change/differ for different portions in the same input/feature map/intermediate data R as processed at the given layer.

In some embodiments, the model may comprise runs of layers that implement attention mechanisms AM, alternating with runs of fully connected layers in some *seq2seq*- models, or at least in those with sequential output, For example, in textual processing, the *n* (>=1)-final layer(s), such as the output layer and the (one or more) layers preceding it, implement a fully connected NN.

As briefly mentioned above, in some embodiments of sequence-to-sequence (seq2seq)-processing as envisaged herein, the architecture of model M is of the encoder-decoder type. Such architecture type may comprise two subsystems, one downstream the other, commonly referred to herein as the encoder ENC and the downstream de-coder DEC, downstream of encoder ENC. In particular, the natural language model NL part (there may be additional part(s) ) of the model M may have such an encoder-decoder setup.

Broadly, the de-coder DEC receives output from the encoder ENC, and processes this into the desired output p. Generally, the encoder is operative by using a number of layers including, one or more attention mechanism implementing layers AM, to produce a hidden or latent representation $\kappa$. This representation is thought to encapsulate all, or at least all relevant, information from the input data s = $\bar{s}$. One or both, the encoder and decoder, may include runs of alternating layers L, that alternative between attention mechanism layers and fully connected layers. Other arrangements and processing sequencing are also envisaged herein.

The latent representation $\kappa$ may have a lower data dimension than the input data or intermediate data, however, this is not necessarily required. The data dimension (a whole number), is in general a function of the largest number of specifiers (such a indices) needed to address each element/component or other constituent part of the data. For example, in the natural language processing embodiments envisaged herein, the hidden representation may in fact have a higher dimensional structure as the simple linear sequence input s. In this embodiment, or similar embodiments, elements of the latent representation $\kappa$, may have more than a single data dimension to represent, not only for the positional information of the respective tokens $s_j$, but also the attention scores, on how some or each token relate(s) to one or more other tokens, either in the input data, in some intermediate data, or indeed in the output data, in the same layer, or across multiple layers.

Thus, the trackability of such attention score relations across the data's passage through the model, may necessitate more complex structures, with higher data dimension than the input data.

The output data, such as the sought after imaging instruction sequence $p$, is preferably provided in steps, with one element per position t at output OUT.

The output $p_t$ so produced at position t may depend on one or more prior produced outputs for different positions $p_{t'}$, $t'<t$, and/or an internal state (eg, attention scores, etc) as needed, and as per the particular modelling.

Indeed, whilst the encoder compresses the initial input s via the intermediate representations R into the latent state $\kappa$, the de-coder DEC does the opposite. For example, the de-coder processes the latent representation by in an auto-regressive manner to produce the output sequence $p$ representative of the image instructions. The processing by decoder DEC may be one of data expansion or contraction, depending on data dimension. Such expansion/contraction may be done by producing output token $p_t$ for a given position $t$, based on prior output of previously output sequences tokens $pt'$, $t'<t$. Masking may be used to ensure that each output $p_t$ is dependent only on earlier tokens with position less than $t$.

In general, the output layer OL may be configured to produce as output, a probability distribution over a prior defined vocabulary referred to as dictionary D, such as a medical vocabulary, or otherwise domain specific or general as needed. The dictionary D is a set of tokens from which the output is built. The output $p$ may have pre-set overall length of output strings ($p_t$). Dictionary may include some or all n-grams, such as for n=2, over an alphabet.

Thus, for each output sequence position $t$, a probability distribution over the pre-defined token dictionary D will be output by model M. Specifically, based on the probability distribution $P^t$ at $t$, the token $p_t$ at $t$ is then computed. For example, in some instances, but not all, the token $p_t$ in the dictionary with the highest probability mass according to the computed probability distribution is output for the current position $p_t$. However, in some other embodiments also tokens of lesser probability are considered, and these may be then combined with the highest probability token, as per some selection policy, to produce the final output for the current position, as needed. Thus, output may not always be the token which commands the highest probability, depending in policy. The probability distribution $P^t$ per position t may be understood as a conditional probability distribution, conditioned on one or more tokens $p_{t'}$ at earlier positions $P^t(p_t|t'<t)$.

In addition, in other embodiments, instead of using the de-coder DEC as a downstream component (of the encoder ENC) that produces the natural language presentation from the hidden state $\kappa$, another downstream component (model) DS may be used, such as a diffusion model, or any other model of the generative type such, as a GAN sub-model, or any other, such as VAE, etc. The model is called "downstream" herein as it is downstream the language model LM output, and such model DS accepts langue model's output as its input. The task of the downstream component DS may be to process or further process (to "re-formatting") the latent output $\kappa$ of the encoder into the requested format of imaging instruction, such as in tabular format, or in XML, or in other structured language format, different from natural language if needed.

Reformatting into such machine code may allow directly fed such output into the control interface CI of the imaging apparatus to so cause the machine settings and/or articulating through transducers the patient instructions. As an alternative ML construction, the above-mentioned diffuser DS, or other generative sub-model, may be used in addition to the decoder, as a downstream component of the decoder. Thus, model M may comprise an encoder-autoencoder, followed by the downstream component DS.

The model M may use residual connections (also called skip connections), which help avoid vanishing gradient problem, especially during training.

More generally, and as envisaged herein, is a two-stage model setup: upstream, as a first stage, is the language ML model LM, such as the encoder ENC and/or decoder DEC with (self)attention AM as described above or any other, and with the second stage the downstream model DS, appended to the output of language model LM.

The downstream model DS appended to language model LM, takes the output of the language model LM and generates the suitable output p actually wanted, with format ofp (machine instructions $p_m$ or patient guidance instructions $p_p$) enforced through the second (the downstream) model DS.

For example, the said diffusion model receives the output of the language LM. The language model LM translates user query RQ, e.g. in free-text format or speech, into a parametrization of the output RQ, which is native to the language LM model (eg, free-text output) or some intermediate data R, such as the latent space representation $\kappa$. This language model output p', R, $\kappa$ can then be the input into the second generative model DS. The second generative model DS may implement stable diffusion, or other diffusion model as needed. The second stage downstream model DM produces, based on the language model output LM, the final imaging instruction p.

The format of $p$ could be any data (free text, structed text, tables etc), other than spatially correlated data such as image data. Thus, $p$ is in general not image data, in case of machine-sided output $p_m$. However, when it comes to the patient-sided embodiment $p_p$, the output of diffuser or other generative type downstream model DS, such as GAN, may well be video, or audio as instructions for patient behavior control as mentioned earlier.

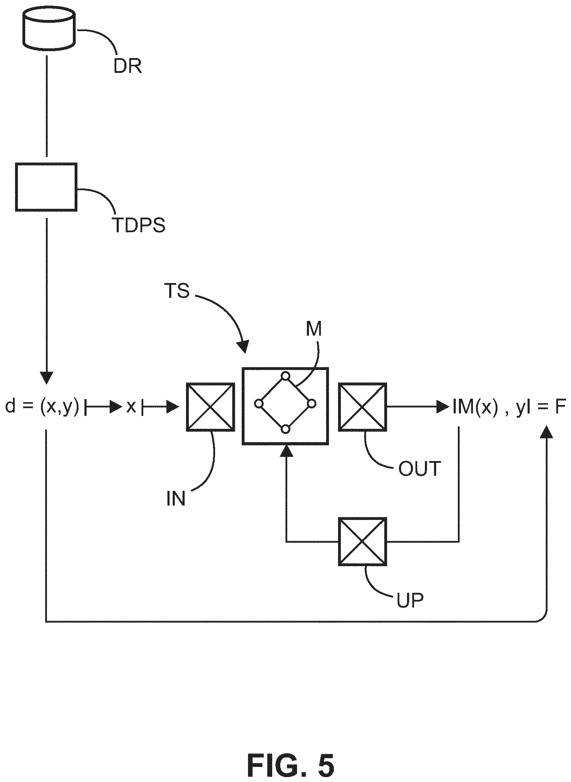

The "primed" notion "$p'$" is used hereinabove, for non-final text data, that still needs reshaping/re-transformation by downstream component DS, as opposed to final text data as produced natively by the language model LM on its own, in embodiments where no generative downstream component DS is necessarily needed, as initially described above in connection with Fig. 5.

As a further variant herein, even if the output $p$ is text, this may represent general imaging instruction that can be shaped by downstream component DS into narrower, more specialist, imaging instructions. A cascaded downstream chain of plural downstream model DSj may be used, to progressively produce more and more specialized output $p'^{(j)}$, until the final output $p$ at the requisite specialization level is obtained. However, such narrowing from general to more specialist output information content can be also obtained by training the language model LM on progressively more specialist knowledge representing training data over multiple, chained, pre-training cycles, with one cycle using the training results (populated parameters) from the previous training cycle(s), as mentioned above. Thus, the required output p at the required level of generality (high level or low level) can be produced natively by the language model LM alone, without any further downstream component DS.

As mentioned, the above described encoder-decoder setup is one option for the language model, but other architectures may be used instead. In the two-stage setup M= (LM, DS) as proposed herein, the intermediate output p' may be configured to describe the required imaging instructions more generally (scan type, field of view, anatomy to be scanned and their angles, etc.), whilst the second downstream model DS takes this output as input, and converts p' to more specific instructions p for a certain imager.

Yet another component, an optional conditioner CON then further translates output of model LM, with or without downstream component DS, into the specific vendor and hardware imager model. This hardware-specific translation module CON can be ML-supported, such as in an iterative GAN approach, to be described further below for reinforced training. The conditioner CON may be implemented without an ML, as a databases lookup operation.

Thus, a cascaded model chain M of plural sub-models, that is, the language model LM, with optional one more downstream components DS, and/or optional downstream conditioner CON, is proposed herein in some embodiments, capable for producing outputs p at progressive levels of specificity, with a given model operable to make the respectively received input from an earlier model in up the chain more specific as the data passes through the chain. As a yet further variant, the second (or) more downstream model DS does not have to be ML, but could involve an optimization algorithm or Monte-Carlo methods, etc.

In some embodiments, the system IF may support a "tap"-enabled user interface, where user has the option to "tap off" the intermediate data p' at higher generality further up the model chain, rather than being confined to the final output p. Thus, user can access inter-model intermediate output p', to so find imaging instructions at the right level of generality for user and task at hand. More than one levels of generality may be so harvested by user in some embodiments.

Because of the number of trainable parameters and the in general large amount of (textual) training data used to train such language models LM, such models are sometimes referred to as "large language models" (LLM). More generally however, the language model may LM be a "foundation model", especially in the context of pre-training as envisaged herein with training on data sets with narrowing scope, representative of more specialist knowledge. Thus, such foundation model was trained before on one task, but may then be repurposed by additional training for another, such as herein, for imaging instruction generation.

In other embodiments, instead of using a diffuser model as downstream component DS, such downstream component DS may be a neural network or decision tree, which maps the output of the language/foundation model LM to imaging instructions p.

In another embodiments, second stage DS may be iterative. A (strict) supervised or reinforced learning algorithm (which may or may not be ML based) is used to ensure compliance with imaging hardware IA and/or patient safety and regulations. This could be done in a GAN-style approach, where one generative (creative) model (generator) comes up with a proposed imaging instruction, and the other model (the discriminator) checks if the proposed imaging instruction is appropriate. This or other such reinforced learning setups may be used. Such approves can be left to iterate, until an appropriate solution is found. Thus, in some embodiments of the two-stage setup, proposed herein, the pre-trained foundation model LM can be fine-tuned in the next stage DS by any form of training algorithm, such as self-supervised, supervised or reinforcement training.

In addition, and with more detailed reference to the optional conditioner COD, this may map the output p' into a specific, such as proprietary format, for a given imager make/mode, as needed to so obtain final output p'. Thus, the output $p$ may be in general free text that describes the imaging procedure with the parameters to be used, or in tabular, vector, matrix form, where the machine settings are listed in terms of parameters, that is numbers, or other symbols. The optional conditioner CON may be a look-up feature, such as a LUT, so may not necessarily be implemented in ML. The conditioner CON may be provided as a part of an optional post-processing pipeline, operative on ML generated output of langue model LM, with or without downstream component DS.

In general, a processing component may be said herein to part of the ML implementation if it includes parameters that are being adapted in the training process, that is, are adapted/adjusted based on training data $d$. If such processing component is unaffected as such by such training process, it may be said to not form part of the ML modelling/implementation, but is part of the said post-processing pipeline, which may thus be said to be an "out-of ML" post-processing pipeline.

The overall model M includes parameters indicated as $\theta$ in Fig. 4. Intermediate data is shown as $R,p'$ in Fig. 4. As said, the scores computed by the attention mechanism are soft scores, and form part of the trained parameters $\theta$ that are adjusted in the training based on the training data d. The number of parameters so trained may be in the order often to the power of six, nine, or even higher, as needed. Larger amount of medical textual material may be processed herein to learn a statistical pattern in the domain specific (eg medical imaging) material, which may be textual, image-based, videos (lectures, etc), which allows generating the output text p during deployment. Thus, operation of model, once trained, may be understood as sampling from the learned probability distributions as per the training data.

A cost function $F$ is used by training system TS, based on which parameters $\theta$ are adjusted, possibly over plural iterations, to so arrive at the trained model M.

An initial model $M^\theta$ may be used with randomly assigned parameters, or any other prior assignment, and this model is then trained based on a training data to arrive at a trained or pre-trained model $M^{\theta'}$ having a different set of parameters as per the training by training system TS. Training system TS effects the adjustment in on or more iterations $k$ <- $k+1$, via training function $T^{(k=k+1)}: \theta \xrightarrow{k} \theta'$. The training function $T$ is a functional of the cost (or more generally objective function) F, and the training data $d$, and may thus be written formally as a parameterized functional $T = T^M(F,d)$, given model architecture M.

Referring now in in more derail to pretraining, once the model is (pre-)trained, it may then be trained again, but this time on a different training data set $d' \neq d$, and the parameters are again adjusted, using the set-up described above based on a different or the same training functions to control the iterations. However, this time, the start parameter in the first iteration $k_0$ are the ones $\theta'$ populated in the previous pre-training cycle $M^{\theta'}$. Thus, the next training cycle post pretraining may yield a newly parameterized model $M^{\theta''}$, $T^{(k=k+1)}: \theta' \xrightarrow{k} \theta''$, and so forth in additional optional training cycles. Because the training is based on generations of training data $d -> d' -> d'' -> d^{(m)}...$, that are more and more domain-specific, intended domain specific performance (eg, medical imaging) tends to improve as has been observed. One or more training cycles may be used starting from 1st pre-training and progressing to pre-trained models of different generations, etc as new, preferably more specialist, training data becomes available. Pre-training may be optional. Another performance increase has been observed by enlarging the pool of training data, not merely in size, but in content, with new examples, in new variations, etc, over what the model M has seen before in prior generation data $d^{(m')}$, $m' < m$.

Fig. 5 shows a schematic diagram of the training system TS. In general, ML proceeds on two or, optional, three phase in the following order: training phase, then testing (generally with several iterations until satisfactory), and finally deployment.

In the training phase, model is trained on data. This may involve multiple training cycles, including pre-training setup as described above. Once trained, test data (different from training data) is used to evaluate performance of model. If error rates are acceptable, model is released for deployment, such as for use to serve intended purpose for which it was trained/tested for. In the instant setting, such use/deployment is to assist the medical user/professional in operating an imager in day to day clinical practice. The data to which model is exposed in deployment is in general different from training and test data. The expectation is that by the training process the model M has "learned" underlying patterns in the data that allows the model to suitable generalize when processing hitherto "unseen", new, data RQ, and still produce useful (reliable) results p, on which medical imaging user can act on in clinical practice.

It is an object of the training system TS to optimize, and hence adapt, the parameters $\theta$ based on the training data $d=\{(x,y)\}$. In other words, the learning can be formulized mathematically as an optimization scheme where the said cost function $F$ is minimized. although a dual formulation of maximizing a utility function may be used instead.

Assuming for now the paradigm of a cost function $F$, this measures the error incurred between data estimated by the neural network model $M(x)$ and the associated target y, as per some or all of the training data pairs $k$:

$$argmin_\theta F = \sum_k || M^\theta(x_k), y_k || \qquad (4)$$

In eq. (4) and below, function $M()$ denotes the result of the model applied to training input x. As said, the cost function may be based on cross-entropy or Kullback-Leiber divergence or similar. In general, any cost function F is suitable for comparison of probability distributions. Statistical techniques such as hypothesis testing etc, may also be considered in some implementations, such as t-tests, ANOVA (Analysis of Variance), chi-square tests, Kolmogorov-Smirnov tests, etc. To be sure, the ML cost function F at (1) is different from, and not related to, the reconstruction cost function q mentioned above at eqs (1)-(3). The index $k$ in (4) relates to different samples/instants $k$ of training data,

In general, there is an error between this output $M(x_k)$ and the associated target $y_k$ of the presently considered $k$-th training data pair. An optimization scheme such as backward/forward propagation or other gradient based methods may then be used to adapt the parameters $\theta$ of the model M so as to decrease the error for the considered pair $(x_k, y_k)$ or for a subset of training pairs from the full training data set. The whole of the training data set may be used at once, or model proceeds in batches over subsets of training data. Each batch uses as the respective starting point the parameter learned from previous batch. In some instances, no summation is needed in (4).

After one or more iterations in a first, inner, loop in which the parameters $\theta$ of the model are updated by updater UP for the current batch (set) of training data (pairs) $(x_k, y_k)$, the training system TS enters a second, an outer, loop where a next training data pair batch $x^{k+1}$, $y^{k+1}$ is processed accordingly. The structure of updater UP depends on the optimization scheme used. For example, the inner loop as administered by updater UP may be implemented by one or more forward and backward passes in a forward/backpropagation algorithm. While adapting the parameters, the aggregated, for example summed, residues of all the training pairs in the current batch is considered, to improve the objective function.

The training system as shown in Fig. 5 can be considered for all learning schemes, in particular supervised schemes. Unsupervised, or self-supervised learning schemes may also be envisaged herein in alternative embodiments. GPUs or other CPUs with multicore design may be used to implement the training system TS, suitably parallelized.

As briefly mentioned above, the training data d may be drawn from historic training data DR as may exist but may also be artificially generated using generative machine learning models in its own right. The training data may be provided by a computing arrangement, the training data provider system TDPS, that may function as a database query facility or, as a suitably trained generative type ML model, such as the said GAN ("generative adversarial network"). See *I Goodfellow et al's* eponymous paper, *"Generative Adversarial Network"*, published on the arXive repository, reference code arXiv: 1406.2661v1 [stat.ML] (2017), available online at https://arxiv.org/abs/1406.2661.

Turning now to the training process in more detail, this is administered by the training system TS. The training system operates an ML training algorithm. The algorithm adjusts the model parameters $\theta$ of the model $M^{\theta}$, based on the cost function $F$. The cost function $F$ processes the training data $d$ to return values in the reals ($\mathbb{R}$). These values, or the behavior of the function $F$ more generally, are/is evaluated by the updater function UP, and the parameters are adjusted, based on such $F$-values/behavior. The exact operation of the updater UP (different and not related to earlier mentioned updater U for reconstruction) depends on the precise numerical algorithm used to solve an optimization problem.

For the proposed medical imaging set-up, the pre-training may be done on a general-purpose textual material, such as may be harvested from the world wide web, online dictionaries, medical journals, websites, reference text books in digital form, etc.

Then, in a second (or more) subsequent training cycle after the pre-training, where the model has been adjusted to a given state $\theta'$, the model may then be re-trained after a post pre-trained cycle on more specific corpus of text, such as medical text on anatomy, physiology, etc. Then, in a third yet more specific training cycle, a text corpus on medical imaging is then used. Lastly and optionally, this pre-training cycle may be complemented by a further treatment cycle which uses specific manufacturer's operation manuals or handbooks of imagers for different makes and models, model years, country specific releases, etc.

In this manner, the training model learns medical knowledge needed for safe and efficient imaging as encapsulated in the corpus of medical knowledge, and then medical imaging corpus in particular. Training data d may comprise the mentioned pairs $\{(x,y)=(x_i, y_i)^i\}$ of training different training input data $x$ and the respective associated target $y$. Index *"i"* as in (4) indicates different instances/samples of pairs, but this indexed notation is mostly dropped herein for simplicity, with $(x,y)$ and similarly "$x$", and "$y$" being a generic reference to such training data pair or its components, the input and target, respectively.

Whilst the building of training data pairs from training inputs x and targets y may be obtained in principle by manual annotation by a domain expert, this may not be feasible when training the model on large training corpuses. Thus, in preferred embodiments, the training system TS is configured for self-supervised or semi-supervised learning.

In self-supervised setups, the pairs are generally $(x,y)$ are found automatically, for example by above repeated mentioned masking procedure as applied to the textual materials. The empty space (white space) may be part of the token dictionary.

Thus, the training data provider system TDPS can automatically generate a potentially large number of such pairs, which are making up the training data set d on which the training system TS, using the considered training algorithm, trains the model M. The training algorithm may be based on back-propagation, or other. The numerical methods used to solve the optimization problem (4) may be gradient-based, such as Gradient Descent, Stochastic Gradient, Nelder-Mead, Newton-Raphson, or any other.

The cost function F itself may be configured to compare probability distributions as has been said above, that are defined over the dictionary D. Suitable such cost functions that capable of comparing probability distributions may include weighted Euclidean distance, or, more preferably, the said cross-entropy, or Kullback-Leibler distance, or other still based on statistical theory.

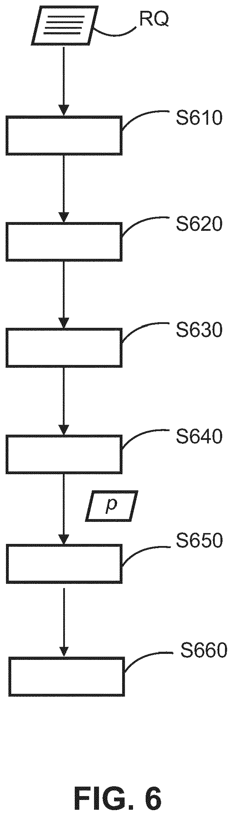

Reference is now made to Fig. 6, which shows a flow chart of a method of controlling or facilitating an imaging operation as envisaged herein in embodiments, based on a trained machine learning model as described above in the previous Figures and an in any of the related embodiments. However, it should be noted that the below described steps are not necessarily tied to the architectures described above, and the method may be understood as a teaching in its own right.

At step S610 an imaging request RQ is received from the user in text, audio (speech), or any other format provided through a suitable interface. The request may be purely "phenomenological", thus may merely define the patient and the purpose of the imaging. The request may be preferably, for better ergonomics, be formulated in speech or in tree text, such as when framing a question for a search engine query, or when putting questions to a chatbot, etc.

At step S620 the input may be pre-processed such as a tokenized, as needed.

At step S630 the optionally tokenized input may be suitably represented as a an embedding in an embedding space, such as one or more vectors as described above.

At step S640 the trained machine model of any of the above embodiments is applied to the input RQ to produce output $p$. The output $p$ represents imaging instructions, either machine-sided or patient-sided, or both, as needed.

At step S650 the output $p$ is the provided for consumption.

For example, at step S660 the so produced and provided imaging instruction(s) p may be used in an imaging procedure.

At a next step S670, the imaging procedure is then carried out, based on the settings/instruction(s) p, to so produce imagery by imager IA at acceptable quality IQ. For example, the machine-sided imaging instructions are fed into the imager IA through suitable control interfacing, such as by interfacing with the native operation control via suitable APIs. The suitable and applicable APIs may thus be accounted for the in the ML produced instruction, The machine learning model herein is either set up for machine-sided or patients-sided imaging instructions. However, a combined model that can do both is not excluded herein. Patient-sided instructions may be conveyed suitably to user and/patient, by text, sounding out, video. Patient-sided instructions may be brought to user's attention such as by displaying, etc. User may then read out the instructions to patient or may use this information to guide patient to assume correct pose and/or exhibit other intended behavior.

As mentioned earlier, the model may comprise a bank of models, where each model of the bank of models is dedicated to a specific imaging modality of a specific type, make or model as needed. Alternatively, the model is trained on a corpus of data that refers to different imager types of different makes and/or models. Thus, the single model may be able to natively provide the tailored imaging instructions for a given imager make/model. More fundamentally, the imaging instruction may even suggest as suitable imaging modality. More than one request may be put by user to the model to so obtain different aspects, details for the intended imaging task.

If the imaging instruction is patient-sided, the input may include additional sensor data, such as imagery acquired by a sensor SEN present in the examination room. This allows to capture imagery, or other data, of the patient's current pose or state of patient, such as controllable/influenceable bio-activity, including breathing, or not, etc, as needed. This is then processed by the machine learning model to produce the imaging instructions for the patient to change their behavior, activity or pose, as needed.

The sensor SEN may be a video camera, depth sensing camera, or any other. It is preferably based on non-ionizing radiation. In particular, this sensor may be integrated in an imaging apparatus such as in the gantry, bore, etc. Instead, sensor SEN may be arranged outside in the imaging exam room, as needed. The sensor is in particular different from the signal source SS used for imaging by the imaging apparatus. However, in some embodiments, the sensor providing the contextual imagery in relation to the patient's pose or bio-activity may in fact be the signal source of the imager. For example, in CT, such context imagery may be a low dose scout image as can be obtained in certain CT imagers. In this manner, a 2D, or even a 3D, scout image may be obtained and used to check pose/bio-activity, instead of using a separate ("out-of-imager") optical or other imaging/sensor device SEN. The sensor is not necessarily (only) image-based, but may instead, or in addition, be arranged as a photo-diode switch, pressure sensor, weight scales, etc, to check correct patent positioning.

If the imaging instruction is machine-sided, such includes parameters/settings/specifications for imaging geometry controlling components, and or DAQs components, such as a detector device, signal source SS, or any other components of the imaging pipeline as described above at Fig. 2.

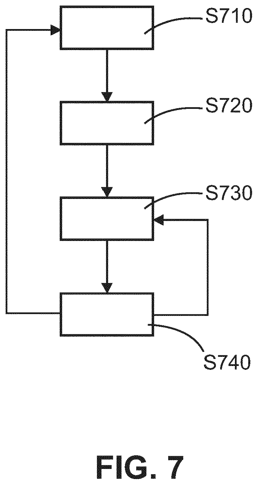

Reference is now made to Fig. 7 which shows a flow chart of a method for training the machine learning model as envisaged herein in embodiments and for user in imaging facilitator IF.

At step S710, training data is accessed or provided. Historical imaging logs, (metadata of) imagery, header data, etc., patient pose imagery, etc, may be accessed for this. For pre-training, a text corpus of a suitable general domain knowledge is provided, such a medical imaging.

Various levels of specialized knowledge data may be considered for multiple cycles of pretraining, such as pretraining on general encyclopedic knowledge, medical knowledge, followed by training on data for medical imaging knowledge, down to imager user manuals, ect.

At step S720, in any given training cycle, the training input $x_k$ is applied to an initialized or earlier trained machine learning model M to produce training output $M(x_k)$.

An error of the training output $M(x_k)$ from the associated target $y_k$ is quantified by cost function F.

One or more parameters of the model are adapted at step S730 and the errors evaluated at S740 in one or more iterations in an inner loop to improve the cost function. For instance, the model parameters are adapted to decrease errors as measured by the cost function. Herein, index $k$ may refer to individual instants/samples, such as pairs, of a given batch (subset) of training data, or the index may refer to the batch as a whole to the whole training data, as needed.

Once sufficient convergence is established, eg by observing the values returned by cost function F (which a real valued function), the training method then returns in an outer loop to step S710, where the next batch of training data pairs is fed in. Forward and/or Backpropagation techniques may be used, or other gradient-based techniques may be used in the inner loop.

In the following various input/output pairings $(RQ, p)$ in deployment phase, and related training data piars $(x,y)$ for training phase are described as envisaged herein in various embodiments. First, various machine-sided settings $p_m$, are described, followed by patient-sided settings $p_p$.

The machine-sided setting will be separately explained with reference to CT imaging and radiography imaging, such as for chest imaging (projection imaging), or any other. However, this is purely illustrative, and not limiting herein, in particular is not at the exclusion of other imaging apparatus/modalities, such as MRI, SPECT, PET, nuclear imaging in general, or any other such as modalities, such as ultrasound and optical tomographic de-coherence tomography, or any other imaging modality as may be used in (medical) imaging.

Turning now first to a CT or radiography application scenario, the deployment input data RQ is as above described, with output $pm$ machine instructions at the desired level of detail.

The corresponding training inputs $x$ may include any one or more of: i) patient complaints, symptoms, reason for medical visit, etc, or other medical history ii) personal patient information such as bio-data (age, weight, height, sex, implants, etc.) or patient ID with automated database lookup to obtain such data ii). Optionally, the request RQ may include current patient positioning information gathered from said external sensors SEN for example, to adjust the imaging parameters accordingly, before starting imaging.

The training (targets) $y$ may include sourced machine settings at sufficient specificity, as compared to the user-friendly "imaging wish" presented in the input request RQ. The machine settings may be hardware specific for a given model (eg, X-ray or CT, or other such as MRI, US, etc), required to fully reproduce the imaging acquisition. This can be parametrized in direct machine-controlling format, or human-readable settings that could be found in the user interface to control the device.

The above -mentioned (supervised) training data $d=\{x,y\}$ does not necessarily need to be sourced from historical recordings, although thss can still be done. Provisioning /sourcing of patient positioning information may be helpful. The necessary inputs x and outputs y can be taken from medical reports and log files, retrospectively. From this data, it can usually be inferred by a keyword-search, patient records, etc, the imaging purpose of the historical scan.

Reinforcement training may be used. Such may be based on user feedback about the procedure or image results. For example, one could have human medical experts, or automated systems, rate historical images for image quality (IQ), such as signal to noise ratio, contrast to noise, artifacts, overall diagnostic value, etc. This rating information can be used to fine-tune the (pre-)trained model M by constructing supervised learning examples to counteract or avoid past issues. Such could also be enforced by the supervisor algorithm mentioned above in the GAN-style approach, by adding new conditions and rules to avoid past IQ problems from happening.

Turning now to the patient-sided data $p_p$, supervised training may be envisaged herein. The deployment input RQ may include i) live camera and audio (eg, microphone) stream, with patient positioning and patient communication being extracted, such as image or audio segmentation. Contextual data from radar or lidar system SEN may be used for patient tracking. The request may further include, instead of i) or may include in addition ii) an indication of the imaging purpose, such as may be taken from medical exam type and objective, in free text description or in any other form. For example, the report may ask for an MRI, CT, or X-ray examination of the right knee of a patient, etc. The request may further include instead of i), ii), or in addition to any one or more of i), ii), information on iii) imaging geometry settings, such as locations and angulations of different imaging hardware components (e.g., X-ray source SS and/or detector DD). Related outputs $p_m$ may include audio, video or textual instructions on how patient should position themselves and/or instructions on bio-activity (breath-hold, or not, etc).

The corresponding supervised training data $(x,y)$ may include historical recordings of the above mentioned kinds of inputs $RQ, p_p$, such as recorded instructions in audio or video, or indeed written recordings, of the positioning or bio-activity instructions, given to patients during historical examinations. This forms target data $y$. Such recordings record positioning/behavior guidance that was administered by human experts trained in the task. The audio recordings may need to be time-aligned with the contextual spatial input data, as training input data x, recorded during the session that capture a stream or still snapshot data of the then-current patient pose in respect of which the instructions y were given.

The model M may be (pre-)trained as a chatbot to provide the output in terms of responses to certain patient questions or problems on positioning. Thus, training data may include recordings of patients being guided by clinical staff for pose correction and/behavior more generally.

The general semantic understanding obtained in unsupervised training should help the model, in particular its language sub-model LM, to generalize from such examples to never-before seen cases, including patients with new positioning concerns, or complying issues.

Thus, in general, in case of patient-sided imaging instructions, a live stream of patient position/pose measurements x acquired by one or more sensors SEN are compared against, preferably time-aligned, historical or synthesized data streams (video, audio, imagery) of expert guidance for related patient behavior. The guidance may be corrective or confirmatory. A suitably balanced proportion of both should be reflected in the training data set. If "real" historical such recordings are hard to come by, such guidance stream may be synthesized by a GAN or other generative type model. The training establishes a relationship between such positional/pose measurement and related patient instructions guidance. Then, in deployment, when model M is presented with such positional measurements, suitable guidance $p_m$ is ML-generated.

The components of the imaging facilitator IF may be implemented as one or more software modules, run on one or more general-purpose processing units PU such as a workstation associated with the imager IA, or on a server computer associated with a group of imagers.

Alternatively, some or all components of the imaging facilitator IF may be arranged in hardware such as a suitably programmed microcontroller or microprocessor, such an FPGA (field-programmable-gate-array) or as a hardwired IC chip, an application specific integrated circuitry (ASIC), integrated into the imaging system IA. In a further embodiment still, the imaging facilitator IF may be implemented in both, partly in software and partly in hardware.

The different components of the imaging facilitator IF may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

**Claims**

1. System for facilitating imaging operation of a medical imaging apparatus, comprising:

   an input interface (IN) capable for receiving input data pertaining to an intended imaging operation in relation to a patient;
   a trained machine learning model (M) having natural language processing capability and capable of processing the input data into output data indicative of an imaging instruction for the said imaging operation, and
   an output interface (OUT) capable of providing the output data, thereby facilitating the imaging operation.

2. System of claim 1, wherein the imaging instruction is any one of i) machine-sided and ii) patient-sided,

   wherein the machine-sided imaging instruction includes one or more machine settings based on which one or more components of the imaging apparatus are controllable to thereby effect, at least in parts, the said imaging operation, and
   wherein the patient-sided imaging instruction includes one or more instructions for the patient behavior in relation to the imaging operation.

3. System of claim 1 or 2, wherein the said patient-sided instruction includes one or more instructions for patient to assume a pose relative to acquisition equipment (SS, DD) of the imaging apparatus (IA), and/or for patient to perform a bio-activity in a particular manner.

4. System of any one of preceding claims, the output interface capable of interfacing with circuitry (CI) of the imaging apparatus thereby facilitating the controlling of the said one or more components.

5. System of any one of preceding claims, wherein model is of the generative type.

6. System of any one of preceding claims, wherein the model is capable of sequence-to-sequence processing.

7. System of any one of preceding claims, wherein the model includes an attention mechanism (AM) capable of coding for context as per the input data and/or with respect to intermediate data (R).

8. System of any one of preceding claims, wherein the input data is indicative of a particular make and model of the imaging apparatus, and wherein the output data is specific to the said particular make and model of the imaging apparatus.

9. A training system (TS) capable of pre-training and/or training, based on respective pre-training/training data, the system's model of any one of the preceding claims.

10. A training data provider system (TDPS) capable of providing the pre-training/training data for the training system of claim 9.

11. Method of facilitating imaging operation of a medical imaging apparatus, comprising:

    receiving (S610) input data pertaining to an intended imaging operation in relation to a patient;
    with a trained machine learning model (M) having natural language processing capability, processing (S640) the input data into output data indicative of an imaging instruction for the said imaging operation, and
    providing (S650) the output data, thereby facilitating the imaging operation.

12. Method of pretraining and/or training, based on respective pre-training/training data, the model in the method of claim 11.

13. Method of providing the training data for the training method of claim 12.

14. A computer program element, which, when being executed by at least one computing system, is adapted to cause the at least one computing system to perform the method as per claim 11, claim 12, or claim 13.

15. At least one computer readable medium having stored thereon the program element of claim 14, or having stored thereon the machine learning model as in any one of the preceding claims.

16. Use of a trained machine learning model capable of natural language processing for generation of at least one imaging instruction for operation of an imaging apparatus for an imaging task.

FIG. 1

**FIG. 2**

**FIG. 3**

**FIG. 4**

**FIG. 5**

RQ

S610

S620

S630

S640

*p*

S650

S660

**FIG. 6**

FIG. 7

Europäisches
Patentamt

European
Patent Office

Office européen
des brevets

# EUROPEAN SEARCH REPORT

**Application Number**

EP 23 21 1490

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/229088 A1 (KONINKLIJKE PHILIPS NV [NL]) 3 November 2022 (2022-11-03) | 1,2,4-16 | INV. G16H40/63 |
| Y | * The whole document, in particular: Page 1 Lines 21 - 22,  Page 8 Line 29 to Page 9 Line 26, Page 15 Lines 18 - 23, Page 17 Line 30 to Page 18 Line 3; and Figures 1, 4 - 11; Claims 1, 2, 5 - 8. *  ----- | 3 | G06N20/00 G16H30/00 G10L15/00  ADD. A61B5/00 |
| X | MORITZ ZAISS ET AL:  "GPT4MR: Exploring GPT-4 as an MR sequence and reconstruction programming assistant", MAGNETIC RESONANCE MATERIALS IN PHYSICS, BIOLOGY AND MEDICINE, vol. 36, 23 September 2023 (2023-09-23), pages S17-S18, XP093147715, GB ISSN: 1352-8661, DOI: 10.1007/s10334-023-01108-9 Retrieved from the Internet: URL:https://link.springer.com/content/pdf/10.1007/s10334-023-01108-9.pdf> | 1,2,4-16 | |
| A | * The whole document, in particular: Pages S17, S18; Figures 1, 2. *  ----- | 3 | |

TECHNICAL FIELDS SEARCHED (IPC)

G16H
G06N
G06F
A61B
G10L

-/--

The present search report has been drawn up for all claims

| Place of search | Date of completion of the search | Examiner |
|---|---|---|
| Munich | 3 May 2024 | Ruschke, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

page 1 of 3

| | Europäisches Patentamt European Patent Office Office européen des brevets | **EUROPEAN SEARCH REPORT** | **Application Number** EP 23 21 1490 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KALRA ANGAD ET AL: "Machine Learning for Automation of Radiology Protocols for Quality and Efficiency Improvement", JOURNAL OF THE AMERICAN COLLEGE OF RADIOLOGY, vol. 17, no. 9, 9 April 2020 (2020-04-09), pages 1149-1158, XP093148271, AMSTERDAM, NL ISSN: 1546-1440, DOI: 10.1016/j.jacr.2020.03.012 Retrieved from the Internet: URL:https://dx.doi.org/10.1016/j.jacr.2020.03.012> | 1,2,4, 8-16 | |
| A | * The whole document, in particular: Abstract, Introduction, Methods; Figures 3 - 5; Tables 2, 5. * ----- | 3,5-7 | |
| X | Thebloke: "Llama-2 7B Chat Model GGUF", , 4 September 2023 (2023-09-04), XP093153274, Retrieved from the Internet: URL:https://huggingface.co/TheBloke/Llama-2-7B-Chat-GGUF [retrieved on 2024-04-17] | 1-16 | **TECHNICAL FIELDS SEARCHED (IPC)** |
| Y | * The language model itself as well as the information given in the model repository, in particular: Model llama-2-7b-chat.Q5_K_M. * ----- | 3 | |
| X | EP 4 252 660 A1 (KONINKLIJKE PHILIPS NV [NL]) 4 October 2023 (2023-10-04) | 1-7,9-16 | |
| Y | * The whole document, in particular: | 3 | |
| A | Paragraphs [0003] - [0005], [0013], [0024] - [0026], [0049] - [0052]; Claims 1, 12. * ----- -/-- | 8 | |

The present search report has been drawn up for all claims

| Place of search | Date of completion of the search | Examiner |
|---|---|---|
| Munich | 3 May 2024 | Ruschke, Stefan |

Europäisches Patentamt

European Patent Office

Office européen des brevets

# EUROPEAN SEARCH REPORT

**Application Number**

**EP 23 21 1490**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MESE ISMAIL ET AL: "Improving radiology workflow using ChatGPT and artificial intelligence", CLINICAL IMAGING, ELSEVIER, NEW YORK, NY, US, vol. 103, 6 October 2023 (2023-10-06), XP087419697, ISSN: 0899-7071, DOI: 10.1016/J.CLINIMAG.2023.109993 [retrieved on 2023-10-06] * The whole document, in particular: Page 3. * | 1-16 | |
| A,D | Vaswani Ashish ET AL: "Attention is all you need", arXiv:1706.03762v1, 12 June 2017 (2017-06-12), XP093106821, Ithaca DOI: 10.48550/ARXIV.1706.03762 Retrieved from the Internet: URL:https://arxiv.org/pdf/1706.03762v1.pdf [retrieved on 2023-11-29] * The whole document. * | 1-16 | TECHNICAL FIELDS SEARCHED (IPC) |

The present search report has been drawn up for all claims

| Place of search | Date of completion of the search | Examiner |
|---|---|---|
| Munich | 3 May 2024 | Ruschke, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 21 1490

This annex lists the patent family members relating to the patent documents cited in the above-mentioned European search report.
The members are as contained in the European Patent Office EDP file on
The European Patent Office is in no way liable for these particulars which are merely given for the purpose of information.

03-05-2024

| Patent document cited in search report | Publication date | Patent family member(s) | Publication date |
|---|---|---|---|
| WO 2022229088 A1 | 03-11-2022 | CN 117223064 A | 12-12-2023 |
| | | EP 4330984 A1 | 06-03-2024 |
| | | JP 2024517656 A | 23-04-2024 |
| | | WO 2022229088 A1 | 03-11-2022 |
| EP 4252660 A1 | 04-10-2023 | EP 4252660 A1 | 04-10-2023 |
| | | WO 2023186551 A1 | 05-10-2023 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. M. MITCHELL,**. Machine Learning. McGraw-Hill,, 1997, 2
- **A. VASWANI**. Attention is all you Need. *arXiv:1706.03762*, 2017, https://arxiv.org/abs/1706.03762
- **J DEVLIN et al.** BERT: Pre-training of Deep Bidirectional Transformers for Language Understanding. *arXiv: 1810.04805v2*, 2018

- **BROWN et al.** Language Models are Few-Shot Learners. *arXiv:2005.14165*, 2020, https://arxiv.org/abs/2005.14165
- Generative Adversarial Network. *arXiv: 1406.2661v1*, 2017, https://arxiv.org/abs/1406.2661